# EUROPEAN PATENT APPLICATION

(11) **EP 1 674 469 A1**
(43) Date of publication of application: **28.06.2006**
(21) Application number: 04090508.5
(22) Date of filing: 22.12.2004
(51) Int. Cl.: C07D 513/08, A61K 31/529, A61P 35/00

(54) **Sulfonamido-macrocycles as Tie2 inhibitors**

(71) Applicant: Schering Aktiengesellschaft, 13353 Berlin (DE)
(72) Inventor: Kettschau, Georg, 13187 Berlin (DE); Briem, Hans, 28279 Bremen (DE); Hartung, Ingo, 30175 Hannover (DE); Lücking, Ulrich, 10245 Berlin (DE); Schäfer, Martin, 13187 Berlin (DE); Thierauch, Karl-Heinz, 14169 Berlin (DE); Schwede, Wolfgang, 16548 Glienicke (DE); Husemann, Manfred, 16540 Hohen Neuendorf (DE)

(57) **Abstract**

The invention relates to sulfonamido-macrocycles according to the general Formula I and the salts thereof, to pharmaceutical compositions comprising the sulfonamido-macrocycles and to a method of preparing the sulfonamido-macrocycles as well as the use thereof for manufacturing a pharmaceutical composition for the treatment of diseases of dysregulated vascular growth or of diseases which are accompanied with dysregulated vascular growth, wherein the compounds effectively interfere with angiopoietin and therefore influence Tie2 signalling. 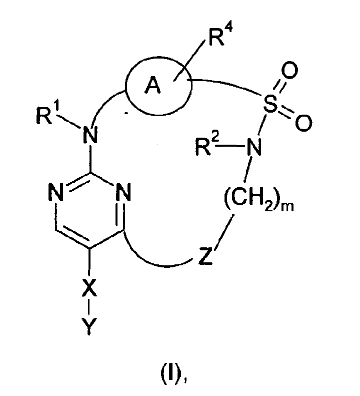 wherein R¹, R², R⁴, A, X, Y, Z and m have the meaning as given in the specification and the claims.

## Description

The invention relates to sulfonamido-macrocycles and the salts thereof, to pharmaceutical compositions comprising the sulfonamido-macrocycles and to methods of preparing the sulfonamido-macrocycles as well as to the use thereof.

In order to defeat diseases with dysregulated vascular growth like cancer different strategies were developed. One possible strategy is the blockade of angiogenesis to the tumour tissue, because tumour angiogenesis is a prerequisite for the growth of solid tumours.

The angiogenesis represents beside the vasculogenesis one of two basic processes during the genesis of vasculature. Vasculogenesis names the neoplasm of vasculature during the embryo development, wherein the angiogenesis describes the neoplasm of vasculature by sprouts or division of present vasculature. It has been found that two receptors expressed on endothelial cells, VEGF- (vascular endothelial growth factor) and Tie-receptors, are essential for normal development of vascular tissue as blood vessels (Dumont *et al.,* (1994). Dominant-negative and targeted null mutations in the endothelial receptor tyrosine kinase, tek, reveal a critical role in vasculogenesis of the embryo. *Genes Dev,* **8**:1897-909; Sato *et al.:* "Distinct roles of the receptor tyrosine kinases Tie-1 and Tie-2 in blood vessel formation" *Nature.* **1995**, Jul 6; 376(6535):70-4.).

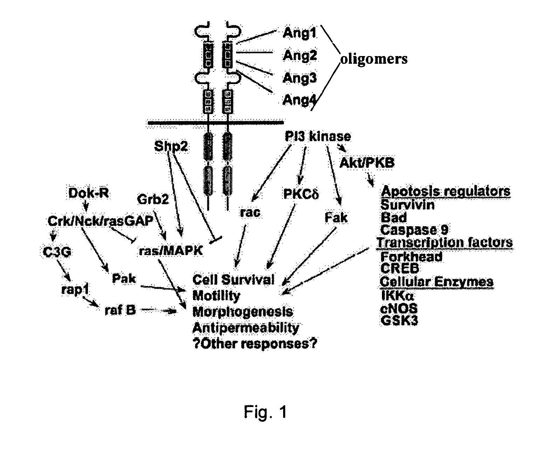

The mechanism of Tie2 signalling was characterized by different searchers, wherein different angiopoietins were found to be involved. So it could be explained that angiopoietin-1 if bound to the extracellular domain of the Tie2-receptor stimulates autophosphorylation and activates the intracellular kinase domain. Angiopoietin-1 activation of Tie2 however does not stimulate mitogenesis but rather migration. Angiopoietin-2 can block angiopoietin-1 mediated Tie2 activation and the resulting endothelial migration. This indicates that angiopoietin-2 is a naturally occurring inhibitor of Tie2 activation (Maisonpierre *et al.:* "Angiopoietin-2, a natural antagonist for Tie2 that disrupts in vivo angiogenesis". *Science.* **1997,** Jul 4; 277(5322):55-60; Witzenbichler *et al*.: "Chemotactic properties of angiopoietin-1 and -2, ligands for the endothelial-specific receptor tyrosine kinase Tie2". *J Biol Chem.* **1998,** Jul 17; 273(29):18514-21). For an overview see Figure 1 modified by Peters *et al.* (Peters *et al.:* "Functional significance of Tie2 signalling in the adult vasculature". *Recent Prog Horm Res.* **2004;** 59:51-71. Review.).

Receptor dimerization results in cross-phosphorylation on specific tyrosine-residues. Receptor cross-phosphorylation has a dual effect: it enhances the receptor's kinase activity and it provides binding sites for signalling molecules possessing phosphotyrosine binding domains (SH2 and PTB domains) (Pawson T.: "Regulation and targets of receptor tyrosine kinases". *Eur J Cancer.* **2002,** Sep, 38 Suppl5:S3-10. Review).

The signalling cross-talk between the PI3-K pathway and the Dok-R pathway is required for an optimal chemotactic response downstream of Tie2. Other recent studies have shown that Tie2-mediated activation of the PI3-K/Akt pathway is required for endothelial nitric oxide synthase (eNOS) activation, focal adhesion kinase activation, and protease secretion, all of which may contribute importantly to Tie2 function during angiogenesis (Kim I. *et al.:* "Angiopoietin-1 regulates endothelial cell survival through the phosphatidylinositol 3'-Kinase/Akt signal transduction pathway". *Circ Res.* **2000,** Jan 7-21; 86(1):24-9; Babaei *et al.:* "Angiogenic actions of angiopoietin-1 require endothelium-derived nitric oxide". *Am J Pathol.* **2003,** Jun; 162(6):1927-36).

For normal development a balanced interaction between the receptors and socalled ligands is necessary. Especially the angiopoietins, which signal via Tie2 receptors, play an important role in angiogenesis (Babaei *et al.,* **2003).**

The broad expression of Tie2 in adult vasculature has been confirmed in transgenic mice using Tie2 promoter driven reporters (Schlaeger *et al.:* "Uniform vascular-endothelial-cell-specific gene expression in both embryonic and adult transgenic mice". *Proc Natl Acad Sci U S A.* 1997, Apr 1; 94(7):3058-63; Motoike *et al.:* "Universal GFP reporter for the study of vascular development". *Genesis.* **2000,** Oct; 28(2):75-81). Immunohistochemical analysis demonstrated the expression of Tie2 in adult rat tissues undergoing angiogenesis. During ovarian folliculogenesis, Tie2 was expressed in the neo-vessels of the developing corpus luteum. Angiopoietin-1 and angiopoietin-2 also were expressed in the corpus luteum, with angiopoietin-2 localizing to the leading edge of proliferating vessels and angiopoietin-1 localizing diffusely behind the leading edge (Maisonpierre *et al.,* 1997). It was suggested that angiopoietin-2-mediated inhibition of Tie2 activation serves to "destabilize" the vessel, to make it responsive to other angiogenic growth factors such as VEGF. Subsequently, angiopoietin-1-mediated activation of Tie2 would trigger stabilization of the neovasculature.

The disruption of Tie2 function shows the relevance of Tie2 for neoangiogenesis in transgenic mice resulting in early embryonic lethality as a consequence of vascular abnormalities (Dumont *et al.,* **1994**; Sato *et al.,* **1995**). Tie2-/- embryos failed to develop the normal vessel hierarchy, suggestive of a failure of vascular branching and differentiation. Tie2-/- embryos have a decreased number of endothelial cells and furthermore less contact between endothelial cells and the underlying pericytes/smooth muscle cells. This implies a role in the maturation and stabilization of newly formed vasculature.

The studies in mice with transgenic or ablated Tie2 gene suggest a critical role for Tie2 in maturation of vascular development in embryos and in adult vasculature. Conditional expression of Tie2 in the endothelium of mice homozygous for a Tie2 null allele partially rescued the embryonic lethality of the Tie2 null phenotype (Jones N *et al.:* "Tie receptors: new modulators of ngiogenic and lymphangiogenic responses." *Nat Rev Mol Cell Biol.* 2001 Apr; 2(4):257-67. Review). Mice lacking functional angiopoietin-1 expression and mice over-expressing angiopoietin-2 both displayed a phenotype similar to Tie2-/- mice (Suri *et al.:* "Requisite role of angiopoietin-1, a ligand for the TIE2 receptor, during embryonic angiogenesis." *Cell.* 1996 Dec 27; 87(7): 1171-80; Maisonpierre PC *et al.:* "Angiopoietin-2, a natural antagonist for Tie2 that disrupts in vivo angiogenesis. *Science.* **1997** Jul 4; 277(5322):55-60.).

Angiopoietin-2 -/- mice have profound defects in the growth and patterning of lymphatic vasculature and fail to remodel and regress the hyaloid vasculature of the neonatal lens (Gale *et al.:* "Angiopoietin 2 is required for postnatal angiogenesis and lymphatic patterning, and only the latter role is rescued by Angiopoietin-1". *Dev Cell.* **2002,** Sep; 3(3):411-23). Angiopoietin-1 rescued the lymphatic defects, but not the vascular remodeling defects. So angiopoietin-2 might function as a Tie2 antagonist in blood vasculature but as a Tie2 agonist in developing lymph vasculature.

Tie2 also plays a role in pathological angiogenesis. It was shown that mutations in Tie2 cause inherited venous malformations and enhance both ligand dependent and independent Tie2 kinase activity (Vikkula *et al.:*
"Dysmorphogenesis caused by an activating mutation in the receptor tyrosine kinase TIE2". *Cell.* **1996,** Dec 27; 87(7):1181-90). Tie2 expression was investigated in human breast cancer tumour specimens and Tie2 expression was found in the vascular endothelium both in normal breast tissue and in breast tumours. The proportion of Tie2-positive tumour microvessels was increased in tumours as compared to normal breast tissue (Peters KG *et al.:*
"Expression of Tie2/Tek in breast tumour vasculature provides a new marker for evaluation of tumour angiogenesis. *Br J Cancer.* **1998,** 77(1):51-6).

Angiopoietin-1 overexpression in tumour models resulted in decreased tumour growth. The effect is possibly related to angiopoietin-1 mediated stabilization of the tumour vasculature, which renders the vessels resistant to angiogenic stimuli (Hayes *et al.:* "Expression and function of angiopoietin-1 in breast cancer". *Br J Cancer.* **2000,** Nov; 83(9):1154-60; Shim *et al.:* 'inhibition of angiopoietin-1 expression in tumour cells by an antisense RNA approach inhibited xenograft tumour growth in immunodeficient mice". *Int J Cancer.* **2001,** Oct 1; 94(1):6-15; Shim *et al.:* "Angiopoietin 1 promotes tumour angiogenesis and tumour vessel plasticity of human cervical cancer in mice". *Exp Cell Res..* 2002, Oct 1; 279(2):299-309; Hawighorst *et al.:* "Activation of the tie2 receptor by angiopoietin-1 enhances tumour vessel maturation and impairs squamous cell carcinoma growth". *Am J Pathol.* **2002,** Apr;160(4):1381-92.; Stoeltzing et al.: "Angiopoietin-1 inhibits vascular permeability, angiogenesis, and growth of hepatic colon cancer tumours". *Cancer Res.* **2003,** Jun 15; 63(12):3370-7.).

Corneal angiogenesis induced by tumour cell conditioned medium was inhibited by recombinant sTie, despite the presence of VEGF. Mammary tumour growth was significantly inhibited in a skin chamber tumour model recombinant sTie2 (Lin *et al.:* "Inhibition of tumour angiogenesis using a soluble receptor establishes a role for Tie2 in pathologic vascular growth". *J Clin Invest.* **1997,** Oct 15;100(8):2072-8; Lin *et al.:* "Antiangiogenic gene therapy targeting the endothelium-specific receptor tyrosine kinase Tie2". *Proc Natl Acad Sci U S A.* **1998,** Jul 21; 95(15):8829-34). Similar sTie constructs have shown comparable effects in different tumour models (Siemeister *et al.:* "Two independent mechanisms essential for tumour angiogenesis: inhibition of human melanoma xenograft growth by interfering with either the vascular endothelial growth factor receptor pathway or the Tie-2 pathway". *Cancer Res.* **1999,** Jul 1; 59(13):3185-91; Stratmann *et al.:* "Differential inhibition of tumour angiogenesis by tie2 and vascular endothelial growth factor receptor-2 dominant-negative receptor mutants". *Int J Cancer.* **2001**, Feb 1; 91(3):273-82; Tanaka *et al.:* "Tie2 vascular endothelial receptor expression and function in hepatocellular carcinoma". *Hepatology.* **2002**, Apr; 35(4):861-7).

When the interaction of angiopoietin-2 with its receptor is blocked by application of a neutralizing anti-angiopoietin-2 monoclonal antibody, the growth of experimental tumours can be blocked efficiently again pointing to the important role of Tie2 in tumour angiogenesis and growth (Oliner *et al.:* "Suppression of angiogenesis and tumour growth by selective inhibition of angiopoietin-2". *Cancer Cell.* **2004,** Nov; 6(5):507-16.) So inhibiting the Tie2 pathway will inhibit pathological angiogenesis.

To influence the interaction between receptor and ligand it could be shown that angiogenesis may be blocked with blockers like Avastin which interfere with VEGF signal transduction to endothelial cells.

Avastin is a clinically effective antibody that functions as tumour growth inhibitor by blockade of VEGFR mediated angiogenic signalling. Thus interference with VEGF signalling is a proven clinical principle. VEGF-C is a molecule inducing lymph angiogenesis via VEGFR 3. The blockade of this signal pathway is inhibiting diseases associated with lymph angiogenesis as is lymphedema and related diseases (Saharinen *et al.:* "Lymphatic vasculature: development, molecular regulation and role in tumour metastasis and inflammation." *Trends Immunol.* **2004,** Jul:25(7): 387-95. Review).

Pyrimidines and their derivatives have been frequently described as therapeutic agents for diverse diseases. A series of recently published patent applications describes their use as inhibitors of various protein kinases, for example WO 2003/032994 A, WO 2003/063794 A, and WO 2002/096888 A. More specifically, certain pyrimidine derivatives have been disclosed as inhibitors of protein kinases involved in angiogenesis, such as VEGF or Tie2, for example benzimidazole substituted 2,4-diaminopyrimidines (WO 2003/074515 A) or (bis)anilino-pyrimidines (WO 2003/066601 A). Very recently, pyrimidine derivatives in which the pyrimidine constitutes a part of a macrocyclic ring system have been reported to be inhibitors of CDKs and/or VEGF (WO 2004/026881 A), or of CDK2 and/or CDK5, respectively (WO 2004/078682 A).

A particular problem in using such known substances as inhibitors or blockers is that their use at the same time is often accompanied with undesired cytotoxic side effects on normal developing and proliferating tissue. This originates from substances which are less selective and at the same time dose tolerability problems.

Therefore the aim of the present invention is to provide compounds, which are useful for the treatment of diseases of dysregulated vascular growth or diseases which are accompanied by dysregulated vascular growth. Furthermore the prior art problems shall be prevented, especially compounds shall be provided, which show low toxic side effects on normal proliferating tissue but are effectively inhibiting endothelial cell migration at small concentrations. This will further reduce undesired side effects.

The solution to the above problems is achieved by providing compounds derived from a class of sulfonamido-macrocycles and the salts thereof in accordance with claim 1, methods of preparing sulfonamido-macrocycles in accordance with claims 21 and 22, a pharmaceutical composition in accordance with claim 23, the use of the compounds in accordance with claim 24 and a method for treating diseases with the compounds in accordance with claim 31.

The application relates to a compound of the general Formula 1: 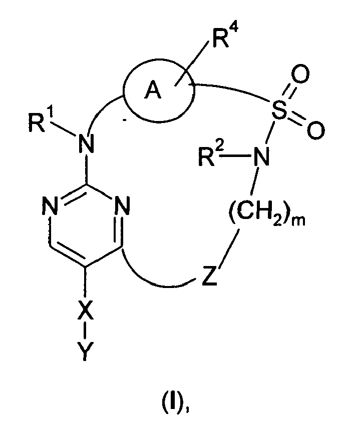 wherein
- A: is phenylene or C₆-heteroarylene;
- Z: is selected from the group comprising O, S, NR³ and CHR³;
- R¹ R² and R³: are the same or different and are independently from each other selected from the group comprising hydrogen and -C₁-C₁₀-alkyl, wherein -C₁-C₁₀-alkyl is unsubstituted or singly or multiply substituted with hydroxy;
- R⁴: is selected from the group comprising hydrogen, halogen, nitro, amino, cyano, -C₁-C₆-alkyl, -C₁-C₆-alkoxy, -NH-C₁-C₆-alkyl, -N(C₁₋C₆-alkyl)₂, -(CH₂)ₚ-COR⁵, -(CH₂)ₚ-NHCOR⁵, -(CH₂)ₚ-NH-CO-NR⁵R⁶, -(CH₂)ₚ-NHS(O)₂R⁵, -(CH₂)ₚ-CO-NR⁵R⁶ and -O-(CH₂)ₚ₋COR⁵,
- X: is a bond or methylene;
- Y: is selected from the group comprising methylenedioxyphenyl, ethylenedioxyphenyl, -phenylene-D-NH-COR⁵, -phenylene-D-NH-CONR⁵R⁶, -phenylene-D-NH-S(O)₂R⁵, -phenylene-D-O-(CH₂)p-COR⁵, -phenylene-D-O-(CH₂)ₚ-R⁵, -N-(R⁵-A)-piperazin-N'-ylmethyl, -N-(R⁷-SO₂-)-piperazin-N' -yl, -oxy-C₆-C₁₈-aryl, -oxy-C₅₋C₁₈-heteroaryl, -oxy-(CH₂)ₙ-NH-COR⁵, -oxy-(CH₂)ₙ-NH-CONR⁵R⁶, -oxy-(CH₂)ₙ-NH-S(O)₂R⁵, -NH-(CH₂)ₙ-NH-COR⁵, -NH-(CH₂)ₙ-NH-CONR⁵R⁶, -NH-(CH₂)ₙ-NH- S(O)₂R⁵ and -phenylene-NH-E-CONR⁵R⁶, wherein phenylene is unsubstituted or singly or multiply substituted independently from each other with hydroxy, halogen, nitro, cyano, carboxy, amino, -C₁-C₆-alkyl, -C₁-C₆-alkoxy, -NH-C₁₋C₆-alkyl, -N(C₁-C₆-alkyl)₂, -C₁-C₆-halogenalkyl, -C₁-C₆₋halogenalkoxy, -C₁-C₆-alkylthio and/or -C₁-C₆-alkylcarbonyl, and wherein -OXY-C₆-C₁₈-aryl and -oxy-C₅-C₁₈-heteroaryl are unsubstituted or singly or multiply substituted independently from each other with hydroxy, halogen, nitro, cyano, carboxy, amino, -C₁-C₆-alkyl, -C₁-C₆-alkoxy, NH-C₁-C₆-alkyl, N(C₁-C₆-alkylh, -C₁₋C₆-haloalkyl, C₁-C₆-haloalkoxy, C₁-C₆-alkylthio, C₁-C₆₋alkylcarbonyl, -NH-S(O)₂-R⁵, -NH-COR⁵, -NHCONR⁵R⁶, -O-(CH₂)ₚ-COR⁵ and/or -O-(CH₂)p-R⁵,
- A: is -SO₂-, -CONH- or -CO-;
- D: is a bond, methylene or ethylene;
- E: is -CR⁸R⁹-, wherein R⁸ and R⁹ are the same or different and are independently from each other selected from the group comprising hydrogen and methyl; or R⁸ and R⁹ together form a 3- to 10-membered methylene tether, in which up to two methylene groups are optionally replaced by O, S and/or NR¹;
- R⁵: is selected from the group comprising hydrogen and residues selected from the group comprising -C₁-C₆-alkyl, -C₂-C₆-alkenyl. -C₂-C₆-alkinyl, -C₃-C₈-cycloalkyl, -(CH₂)ₚ-C₆-C₁₁-aryl and -(CH₂)p-C₅-C₁₀-heteroaryl, wherein said residues are unsubstituted or singly or multiply substituted independently from each other with hydroxy, halogen, nitro, cyano, carboxy, amino, -C₁-C₆-alkyl, -C₁₋C₆-alkenyl, -C₁-C₆-alkinyl, -C₁-C₆-alkoxy, -NH-C₁-C₆-alkyl, -N(C₁₋C₆-alkyl)₂, -C₁-C₆-halogenalkyl, -C₁-C₆-halogenalkoxy, -C₁-C₆₋alkylthio, -S(O)-C₁-C₆-alkyl, -S(O)₂-C₁-C₆-alkyl, -C₁-C₆₋alkylcarbonyl, phenyl, phenoxy and/or pyridyl, and wherein the C-atoms of the C -backbone of -C₃-C₈-cycloalkyl are uninterrupted or singly or multiply interrupted by nitrogen-atoms, oxygen-atoms, sulphur-atoms and/or one or more C=O-moieties and/or wherein one or more double bonds may be contained in the C-backbone; or R⁵ and R⁶ together form a 3- to 10-membered methylene tether, in which up to two methylene groups may be replaced by O, S and/or -NR¹;
- R⁶: is hydrogen or -C₁-C₁₀-alkyl, or R⁵ and R⁶ together form a 3- to 10-membered methylene tether, in which up to two methylene groups may be replaced by O, S and/or NR¹;
- R⁷: is -C₆-C₁₁-aryl or -C₅-C₁₀-heteroaryl, wherein -C₆-C₁₁-aryl or -C₅₋C₁₀-heteroaryl are unsubstituted or singly or multiply substituted with hydroxy, halogen, nitro, cyano, carboxy, amino, -C₁-C₆-alkyl, -C₁-C₆-alkoxy, -NH-C₁-C₆-alkyl, -N(C₁-C₆-alkyl)₂, -C₁-C₆-haloalkyl, -C₁-C₆-haloalkoxy, -C₁-C₆-alkylthio, -S(O)-C₁-C₆-alkyl, -S(O)₂-C₁₋C₆-alkyl, -C₁-C₆-alkylcarbonyl, phenyl, phenoxy and/or pyridyl;
- m: is from 3 to 6;
- n: is 2 or 3;
- p: is from 0 to 2; and
solvates, hydrates, N-oxides, isomers, diastereomers, enantiomers and salts thereof.

The terms as mentioned herein below and in the claims have preferably the following meanings:

The term alkyl preferably refers to branched and unbranched alkyl, meaning e.g. methyl, ethyl, n-propyl, iso-propyl, n-butyl, iso-butyl, *tert-butyl,* sec-butyl, pentyl, iso-pentyl, hexyl, heptyl, octyl, nonyl and decyl and the isomers thereof.

The term alkoxy preferably refers to branched and unbranched alkoxy, meaning e.g. methoxy, ethoxy, propyloxy, *iso*-propyloxy, butyloxy, iso-butyloxy, *tert-*butyloxy, sec-butyloxy, pentyloxy, *iso*-pentyloxy, hexyloxy, heptyloxy, octyloxy, nonyloxy, decyloxy, undecyloxy and dodecyloxy and the isomers thereof.

The term cycloalkyl preferably refers to cycloalkyl, meaning e.g. cyclopropyl, cyclobutyl, cyclopentyl, cyclohexyl and cycloheptyl. Cycloalkyl moieties which are singly or multiply interrupted by nitrogen-atoms, oxygen-atoms and/or sulphur-atoms refer e.g. to oxyranyl, oxetanyl, aziridinyl, azetidinyl, tetrahydrofuranyl, pyrrolidinyl, morpholinyl, dithianyl, thiomorpholinyl, piperazinyl, trithianyl and chinuclidinyl. Cycloalkyl moieties, wherein the C-backbone contains one or more double bonds in the C-backbone refer e.g. to cycloalkenyl, such as cyclopropenyl, cyclobutenyl, cyclopentenyl, cyclohexenyl and cycloheptenyl, wherein the linkage can be provided to the double or single bond.

Halogen preferably refers to fluorine, chlorine, bromine and iodine.

The term alkenyl preferably refers to branched and unbranched alkenyl, e.g. to vinyl, propen-1-yl, propen-2-yl, but-1-en-1-yl, but-1-en-2-yl, but-2-en-1-yl, but-2-en-2-yl, but-1-en-3-yl, 2-methyl-prop-2-en-1-yl and 2-methyl-prop-1-en-1-yl.

The term alkinyl preferably refers to branched and unbranched alkinyl, e.g. to ethinyl, prop-1-in-1-yl, but-1-in-1-yl, but-2-in-1-yl and but-3-in-1-yl.

The term aryl refers to cyclic or polycyclic aromates, e.g. to phenyl, naphthyl and biphenyl.

The term arylene refers to cyclic or polycyclic aromates, e.g. to phenylene, naphahylene and biphenylene.

The term heteroaryl refers to cyclic or polycyclic aromates, e.g. to five-membered heteroaromates, such as thiophenyl, furanyl, oxazolyl, thiazolyl, imidazolyl, pyrazolyl, triazolyl, thia-4H-pyrazolyl and benzo-derivates thereof, or six-membered heteroaromates, such as pyridinyl, pyrimidinyl, triazinyl, quinolinyl, isoquinolinyl and benzo-derivates thereof, e.g. quinolinyl, isoquinolinyl.

The term heteroarylene refers to cyclic or polycyclic aromates, e.g. to five-membered heteroaromates, such as thiophenylene, furanylene, oxazolylene, thiazolylene, imidazolylene, pyrazolylene, triazolylene, thia-4H-pyrazolylene and benzo-derivates thereof, or six-membered heteroaromates, such as pyridinylene, pyrimidinylene, triazinylene and benzo-derivates thereof, e.g. quinolinylene, isoquinolinylene.

The compound according to Formula I (sulfonamido-macrocycles) can exist as N-oxides which are defined in that at least one nitrogen of the compounds of the general Formula I may be oxidized.

The compound according to Formula I (sulfonamido-macrocycles) can exist as solvates, in particular as hydrate, wherein the compound according to Formula I may contain polar solvents, in particular water, as structural element of the crystal lattice of the compounds. The amount of polar solvents, in particular water, may exist in a stoichiometric or unstoichiometric ratio. In case of stoichiometric solvates, e.g. hydrate, are possible hemi-, (semi-), mono-, sesqui-, di-, tri-, tetra-, penta- etc. solvates or hydrates, respectively.

The term isomers refers to chemical compounds with the same number and types of atoms as another chemical species. There are two main classes of isomers, constitutional isomers and stereoisomers.

The term constitutional isomers refers to chemical compounds with the same number and types of atoms, but they are connected in differing sequences. There are functional isomers, structural isomers, tautomers or valence isomers.

In stereoisomers, the atoms are connected sequentially in the same way, such that condensed formulae for two isomeric molecules are identical. The isomers differ, however, in the way the atoms are arranged in space. There are two major sub-classes of stereoisomers; conformational isomers, which interconvert through rotations around single bonds, and configurational isomers, which are not readily interconvertable.

Configurational isomers are, in turn, comprised of enantiomers and diastereomers. Enantiomers are stereoisomers which are related to each other as mirror images. Enantiomers can contain any number of stereogenic centers, as long as each center is the exact mirror image of the corresponding center in the other molecule. If one or more of these centers differs in configuration, the two molecules are no longer mirror images. Stereoisomers which are not enantiomers are called diastereomers. Diastereomers which still have a different constitution, are another sub-class of diastereomers, the best known of which are simple *cis-trans* isomers.

In order to limit different types of isomers from each other reference is made to IUPAC Roles Section E *(Pure Appl Chem* **45,** 11-30, 1976).

The compound according to Formula I (sulfonamido-macrocycles) can exist in free form or in a salt form. A suitably pharmaceutically acceptable salt of the sulfonamido-macrocycles of the invention is, for example, an acid-addition salt of a sulfonamido-macrocycle of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitably pharmaceutically acceptable salt of a sulfonamido-macrocycle of the invention which is sufficiently acidic is an alkali metal salt, for example a sodium or potassium salt, an alkaline earth metal salt, for example a calcium or magnesium salt, an ammonium salt or a salt with an organic base which affords a physiologically acceptable cation, for example a salt with N-methyl-glukamine, dimethyl-glukamine, ethyl-glukamine, lysine, 1,6-hexadiamin, ethanolamine, glucosamine, sarkosine, serinole, tris-hydroxymethyl-aminomethan, aminopropandiole, sovak-base, 1-amino-2,3,4-butantriole.

Compounds of Formula I according to the present invention are particular preferred, wherein
- A: is phenylene;
- Z: is -NR³; and
- m: is 3.

Also compounds are preferred wherein A is pyridinylene.

Further compounds are particularly preferred, wherein
X is a bond and Y is methylenedioxyphenyl or ethylenedioxyphenyl; or
X is a bond and Y is -phenylene-D-NH-COR⁵; or
X is a bond and Y is -phenylene-D-NH-CONR⁵R⁶; or
X is a bond and Y is -phenylene-D-NH-S(O)₂R⁵; or
X is a bond and Y is -phenylene-D-O-(CH₂)ₚ-COR⁵, wherein D is a bond or methylene; or
X is a bond and Y is -phenylen-NH-E-CONR⁵R⁶; or
X is a bond and Y is -oxy-C₆-C₁₈-aryl, wherein -oxy-C₆-C₁₈-aryl is unsubstituted or singly or multiply substituted independently from each other with hydroxy, halogen, nitro, cyano, carboxy, amino, -C₁-C₆-alkyl, -C₁-C₆-alkoxy, -NH-C₁-C₆-alkyl, -N(C₁-C₆-alkyl)₂, -C₁-C₆-haloalkyl, -C₁-C₆-haloalkoxy, -C₁₋C₆-alkylthio, -C₁-C₆-alkylcarbonyl, -NH-S(O)₂-R⁵, -NH-COR⁵, -NHCONR⁵R⁶, -O-(CH₂)ₚ-COR⁵, and/or -O-(CH₂)ₚ-R⁵, preferably -C₆-C₁₈-aryl is phenyl, which may be substituted with -NH-COR⁵ or -NH-CONHR⁵; or
X is bond or methylene and Y is -N-(R⁵-A)-piperazin-N'-ylmethyl; or
X is bond or methylene and Y is -N-(R⁵-SO₂)-piperazin-N'-yl; or
X is a bond and Y is -oxy-(CH₂)ₙ-NH-COR⁵; or
X is a bond and Y is -oxy-(CH₂)ₙ-NH-CONR⁵R⁶; or
X is a bond and Y is -oxy-(CH₂)ₙ-NH-S(O)₂R⁵; or
X is a bond and Y is -NH-(CH₂)ₚ-NH-COR⁵; or
X is a bond and Y is -NH-(CH₂)p-NH-CONR⁵R⁶; or
X is a bond and Y is -NH-(CH₂)ₙ-NH-S(O)₂R⁵; additionally these compounds preferably have A = phenylene; Z = -NR³; and m = 3.

The following compounds are mostly preferred:
Compound 1.1:
   2-[4-[4,4-dioxo-4λ⁶-thia-2,5, 9-triaza-1(2,4)-pyrimidina-3(1,3)-benzenacyclononaphan-1⁵-yl]phenoxy]-1-(4-fluorophenyl)ethanone
Compound 1.2:
   2-[4-[4,4-dioxo-4λ⁶-thia-2,5, 9-triaza-1(2,4)-pyrimidina-3(1,3)-benzenacyclononaphan-1⁵-yl]phenoxy]-1-phenylethanone
Compound 1.3:
   N-[4-[4,4-dioxo-4λ6 -thia-2,5,9-triaza-1 (2,4)-pyrimidina-3(1,3)-benzenacyclononaphan-1⁵-yl]phenyl]-*N*'-phenylurea
Compound 1.4:
   *N-*[4-[4 *,*4-dioxo-4λ⁶-thia-2, 5, 9-triaza-1(2,4)-pyrimidina-3(1,3)-benzenacyclononaphan-1⁵-yl]phenyl]-*N*'-[3-(trifluoromethyl)phenyl]urea
Compound 1.5:
   *N*-[4-[4,4-dioxo-4λ⁶-thia-2,5,9-triaza-1(2,4)-pyrimidina-3(1,3)-benzenacyclononaphan-1⁵-yl]phenyl]-*N*'-[2-fluoro-5-(trifluoromethyl)phenyl]urea
Compound 1.6:
   *N*-[4-[4,4-dioxo-4λ⁶-thia-2,5,9-triaza-1(2,4)-pyrimidina-3(1,3)-benzenacyclononaphan-1⁵-yl]phenyl]-4-methoxybenzenesulfonamide
Compound 1.7:
   15-(1,3-benzodioxol-5-yl)-4-thia-2,5,9-triaza-1(2,4;-pyrimidina-3(1,3)-benzenacyclononaphane 4,4-dioxide

The compounds of the present invention can be used in treating diseases of dysregulated vascular growth or diseases which are accompanied by dysregulated vascular growth. Especially the compounds effectively interfere with angiopoietin and therefore influence Tie2 signalling. Surprisingly the compounds block Tie2 signalling, wherein obviously Tie2 kinase activity is blocked with showing no or very low cell toxicity for cells other than endothelial cells at low concentrations, which is an important advantage over prior art substances. This effect will therefore allow prolonged treatment of patients with the compounds offering good tolerability and high anti-angiogenic efficacy, where persistent angiogenesis plays a pathologic role.

Therefore the compounds of the present invention can be applied for the treatment of diseases accompanied by neoangiogenesis. This holds principally for all solid tumours, e.g. mamma, colon, renal, lung and/or brain tomours and can be extended to a broad range of diseases, where pathologic angiogenesis is persistent. This applies for diseases with inflammatory association, diseases associated with edema of various forms and diseases associated with stromal proliferation and pathologic stromal reactions broadly. Particularly suited is the treatment for gynaecological diseases where inhibition of angiogenic, inflammatory and stromal processes with pathologic character can be inhibited. At the same time the toxic side effects on normal proliferating tissue are low. The treatment is therefore an addition to the existing armament to treat diseases associated with neoangiogenesis.

The compounds of the present invention can be used in particular in therapy and prevention of tumour growth and metastases especially in solid tumours of all indications and stages with or without pre-treatment if the tumour growth is accompanied with persistent angiogenesis. However it is not restricted to tumour therapy but is also of great value for the treatment of other diseases with dysregulated vascular growth. This includes retinopathy and other angiogenesis dependent diseases of the eye (e.g. cornea transplant rejection, age-related macular degeneration), rheumatoid arthritis, and other inflammatory diseases associated with angiogenesis like psoriasis, delayed type hypersensitivity, contact dermatitis, asthma, multiple sclerosis, restenosis, pulmonary hypertension, stroke and inflammatory diseases of the bowel, like Crohn's disease. It includes coronary and peripheral artery disease. It can be applied for disease states like ascites, edema, like brain tumour associated edema, high altitude trauma, hypoxia induced cerebral edema, pulmonary edema and macular edema or edema following burns and trauma. Furthermore it is useful for chronic lung disease, adult respiratory distress syndrome. Also for bone resorption and for benign proliferating diseases like myoma, benign prostate hyperplasia and wound healing for the reduction of scar formation. It is therapeutically valuable for the treatment of diseases, where deposition of fibrin or extracellular matrix is an issue and stroma proliferation is accelerated (e.g. fibrosis, cirrhosis, carpal tunnel syndrome etc). In addition it can be used for the reduction of scar formation during regeneration of damaged nerves, permitting the reconnection of axons. Further uses are endometriosis, pre-eclampsia, postmenopausal bleeding and ovarian hyperstimulation.

A second aspect of the invention is a pharmaceutical composition which contains a compound of Formula I or pharmaceutically acceptable salts thereof, isomers or mixtures of isomers thereof, in admixture with one or more suitable excipients. This composition is particularly suited for the treatment of diseases of dysregulated vascular growth or of diseases which are accompanied with dysregulated vascular growth as explained above.

For using the compounds of the present invention as pharmaceutical product the compounds or mixtures thereof are provided in a pharmaceutical composition, which beside the compounds of the present invention for enteral, oral or parenteral application contain suitably pharmaceutically acceptable organic or inorganic inert base material, e.g. purified water, gelatin, rubber arabicum, lactate, starch, magnesium stearate, talcum, vegetable oils, polyalkylenglycole, etc.

The pharmaceutical composition may be provided in a solid form, e.g. as tablets, dragees, suppositories, capsules or in liquid form, e.g. as a solution, suspension or emulsion. The pharmaceutical composition may additionally contain auxiliary substances, e.g. conservators, stabilisers, wetting agents or emulgators, salts for adjusting the osmotic pressure or buffers.

For parenteral application (including intravenous, subcutaneous, intramuscular, intravascular or infusion) sterile injection solutions or suspensions are preferred, especially aqueous solutions of the compounds in polyhydroxyethoxy containing castor oil.

The pharmaceutical composition may further contain surface active agents, e.g. salts of gallenic acid, phosphorlipids of animal or vegetable origin, mixtures thereof and liposomes and parts thereof.

For oral application tablets, dragèes or capsules with talcum and/or carbonhydrogen carriers and -binders, e.g. lactose, maize and potato starch, are preferred. Further application in liquid form is possible, for example as juice, which contains sweetener if necessary.

The dosage will necessarily be varied depending upon the route of administration, age, weight of the patient, the kind and severity of the illness being treated and similar factors. The daily dose is in the range of 0.5 -1.500 mg. A dose can be administered as unit dose or in part thereof and distributed over the day. Accordingly the optimum dosage may be determined by the practitioner who is treating any particular patient.

Another aspect of the present invention is the method of preparing the compounds according to the present invention.

The following table lists the abbreviations used in this paragraph, and in the Examples section. NMR peak forms are stated as they appear in the spectra, possible higher order effects have not been considered.

| **Abbreviation** | **Meaning** |
|---|---|
| Ac | acetyl |
| Boc | *tert*-butyloxycarbonyl |
| br | broad |
| c- | cyclo- |
| Cl | chemical ionisation |
| d | doublet |
| dd | doublet of doublet |
| DCM | dichloromethane |
| DIPEA | N,N-diisopropylethyl amine |
| DMF | N,N-dimethylformamide |
| DMSO | dimethyl sulfoxide |
| eq. | equivalent |
| ESI | electrospray ionisation |
| GP | general procedure |
| m | multiplet |
| mc | centred multiplet |
| MS | mass spectrometry |
| NMR | nuclear magnetic resonance spectroscopy |
| POPd | dihydrogen dichlorobis(di-tert-butyl phosphinito-κP)palladate(2); CombiPhos Catalysts, Inc. |
| q | quartet |
| s | singlet |
| t | triplet |
| TEA | triethylamine |
| TFA | trifluoroacetic acid |
| THF | tetrahydrofurane |

The following schemes and general procedures illustrate general synthetic routes to the compounds of general Formula I of the invention and are not intended to be limiting. Specific examples are described in the subsequent paragraph. Thus, the compounds of the invention can be prepared starting from halogenated macrocycles **(A)** by palladium catalysed coupling reactions such as Suzuki, Heck, or Sonogashira couplings, or by amination methods well known to those skilled in the art.

One first reaction scheme is outlined herein below: 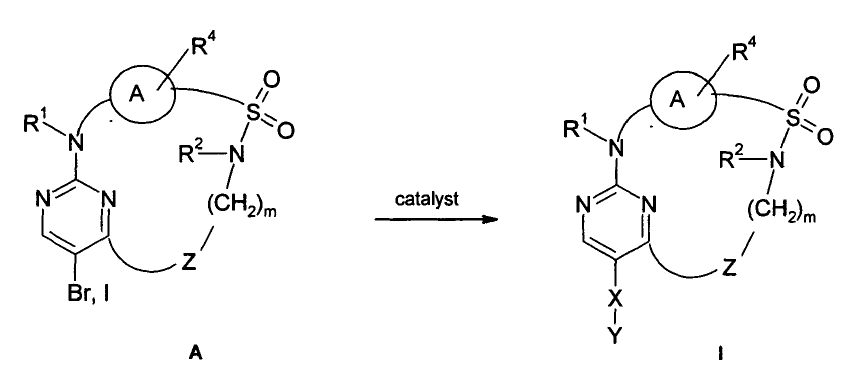 *Scheme 1: Coupling, e.g. Suzuki or Ullmann coupling of macrocycles A, wherein A in macrocycles A is preferably phenylene, Z is -NR*^{*3*} *and m=3, wherein in Formula I X is a bond and Y is e.g. -phenylene-D-NH-COR*^{*5*}*, -phenylene-D-NH-CONR*^{*5*}*R*^{*6*}*, -phenylene-D-NH-S(O)*_{*2*}*R*^{*5*}*, -phenylene-D-O-(CH*_{*2*}*)*_{*p*}*-COR*^{*5*}*, -oxy-C*_{*6*}*-C*_{*11*}*-aryl, or-oxy-C*_{*5*}*-C*_{*10*}*-heteroaryl with the meaning above.*

A second reaction scheme is outlined herein below: 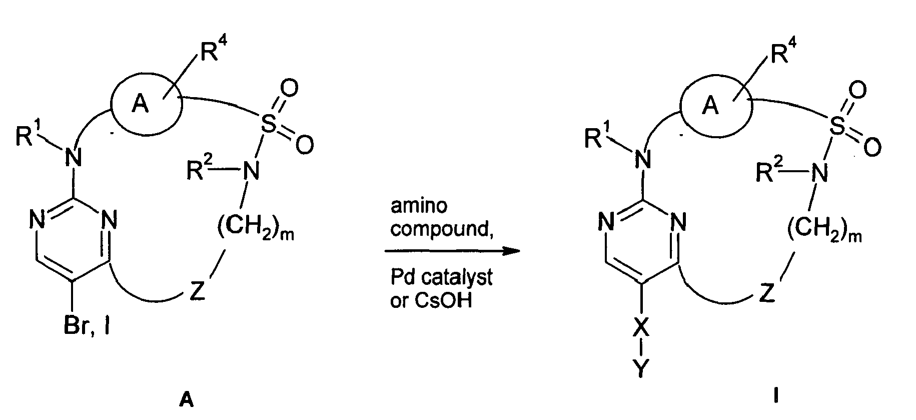 *Scheme* 2: *Amination of macrocycles A, wherein A in macrocycles A is preferably phenylene, Z is -NR*^{*3*} *and m=3, with X-Y, wherein in Formula I X is a bond and Y is e.g. -NH-(CH*_{*2*}*)*_{*n*}*-COR*^{*5*}*-, -NH-(CH*_{*2*}*)*_{*n*}*-CONR*^{*5*}*R*^{*6*}*, or -NH-(CH*_{*2*}*)*_{*n*}*-S(O)*_{*2*}*R*^{*5*} *with the meaning above.*

The synthesis of macrocycles **A** is described in WO 2004/026881 A. Appropriate coupling partners are either commercially available or can be prepared by simple standard functionalisation procedures as shown in Scheme 3 well known to those skilled in the art: 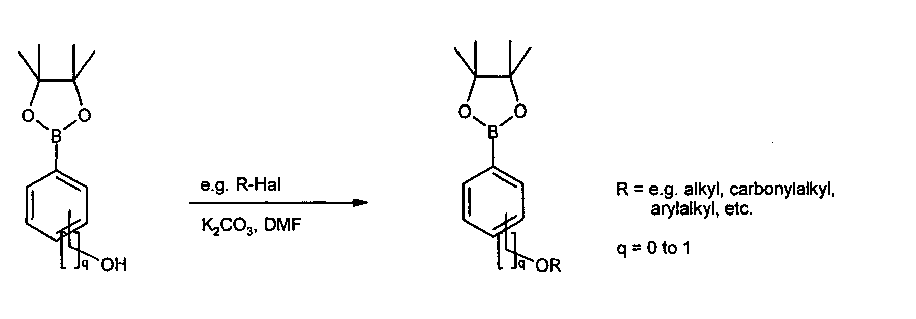 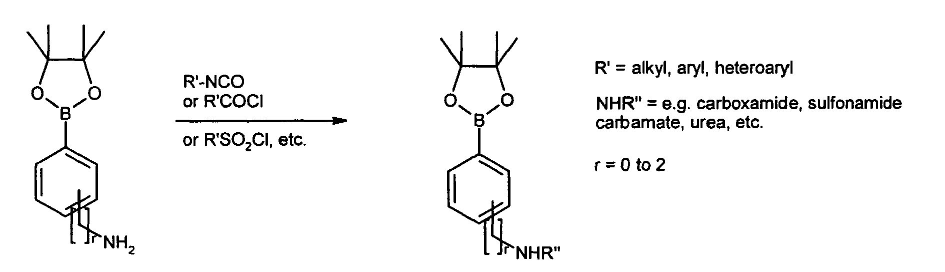 *Scheme* 3: *Synthesis of building blocks for Suzuki couplings*

### General procedure (GP) 1: Alkylation of hydroxyphenylboronic acid esters

A solution of the respective hydroxyphenylboronic acid ester in DMF (4 mL per mmol) was treated with K₂CO₃ (1.2 eq.), followed by the respective halide (1.1 eq.) under an atmosphere of nitrogen. The resulting mixture was stirred for 3 h at room temperature and was then evaporated. The residue was partitioned between ethyl acetate and water, and the organic layer was dried and concentrated. The crude residue was subjected to column chromatography to give the pure products.
Typically, the reactions were run on a 0.5 to 1 mmol scale.

### GP 2: Reaction of aminophenylboronic acid esters with isocyanates

A solution of the respective aminophenylboronic acid ester in DCM (5 mL per mmol boronic ester) was treated with the respective isocyanate (1.05 eq.), followed by TEA (1.1 eq.) at room temperature under an atmosphere of nitrogen. The resulting mixture was stirred overnight and then analysed by TLC. If the reaction did not reach completion after 20 h, additional reagents (isocyanate, 0.26 eq.; and TEA, 0.28 eq.) were supplemented and stirring was continued until the reaction was complete according to TLC. The mixture was evaporated and then subjected to column chromatography. In some cases, the intermediates obtained were still impure and used without further purification. Typically, the reactions were run on a 0.5 to 1 mmol scale.

### GP 3: Sulfonylation of aminophenylboronic acid esters

A solution of the respective aminophenylboronic acid ester in DCM (5 mL per mmol boronic ester) was treated with the respective sulfonyl chloride (1.05 eq.), followed by pyridine (1.1 eq.) at room temperature under an atmosphere of nitrogen. The resulting mixture was stirred overnight and then evaporated, followed by column chromatography of the crude residue to give the pure sulfonamide intermediate.
Typically, the reactions were run on a 0.5 to 1 mmol scale.

### GP 4: Suzuki coupling

A solution of the respective macrocyclic halide in DMF (8 mL per mmol halide) was treated with the respective organoboron compound (1.25 eq.), K₂CO₃ (2.5 eq., either as a solid or as 2 M aqueous solution), and POPd (2.5 mol-%) at room temperature. The stirred resulting mixture was heated to 100 °C. The reaction was monitored by TLC, and additional portions of POPd (2.5 mol-%) and if consumed by then organoboron compound (1.25 eq.) were added. Stirring was continued for another 2 h, and addition of reagents, followed by 2 h stirring at 100 °C was repeated until the macrocyclic halide was completely consumed. After cooling to room temperature, water was added and the resulting suspension was stirred for 30 min. The crude product was isolated by vacuum filtration, dried *in vacuo,* and purified by column chromatography, followed by trituration with methanol, and / or preparative HPLC (e.g. YMC Pro C18RS 5µ, 150 x 20 mm, 0.2% NH₃ in water/acetonitrile) to give the pure coupling products.
Typically, the reactions were run on a 0.25 mmol scale.

### Example compounds

### Preparation of intermediates

### Intermediate 1:

### Preparation of 1-phenyl-2-[4-(4,4,5,5-tetramethyl-1,3,2-dioxaborolan-2-yl) - phenoxy] ethanone

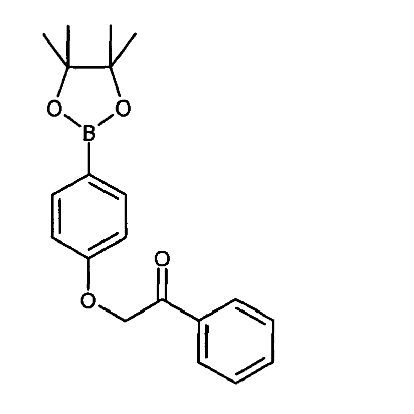

Intermediate 1 was prepared according to GP 1 from 4-(4,4,5,5-tetramethyl-1,3,2-dioxaborolan-2-yl)-phenol and ω-bromoacetophenone. Yield: 67%.
¹H-NMR (CDCl₃, 300 MHz): 8.01 (d, 2 H); 7.74 (d, 2 H); 7.61 (t br, 1 H); 7.49 (t, 2 H); 6.93 (d, 2 H); 5.29 (s, 2 H); 1.31 (s, 12 H).
MS (Cl): [M+NH₄]⁺ = 356.

### Intermediate- 2:

### Preparation of 1-(4-fluorophenyl)-2-[4-(4,4,5,6-tetramethyl-1,3,2-dioxaborolan-2-yl)phenoxy]ethanone

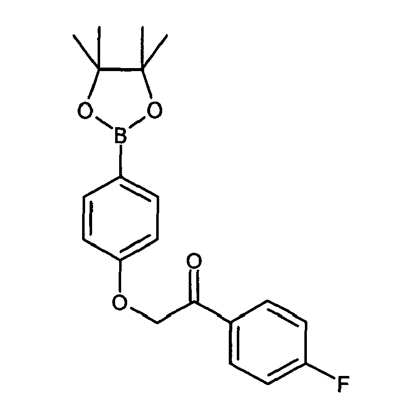

Intermediate 2 was prepared according to GP 1 from 4-(4,4,5,5-tetramethyl-1,3,2-dioxaborolan-2-yl)-phenol and 4-fluorophenacyl bromide. Yield: 66%.
¹H-NMR (CDCl₃, 300 MHz): 8.06 (mc, 2 H); 7.75 (d, 2 H); 7.16 (t, 2 H); 6.92 (d, 2 H); 5.23 (s, 2 H); 1.32 (s, 12 H).
MS (ESI): [M+H]⁺ = 357.

### Intermediate 3:

### Preparation of N-[2-fluoro-5-(trifluoromethyl)phenyl]-N' -[4-(4,4,5,5-tetramethyl-1,3,2-dioxaborolan-2-yl)phenyl]urea

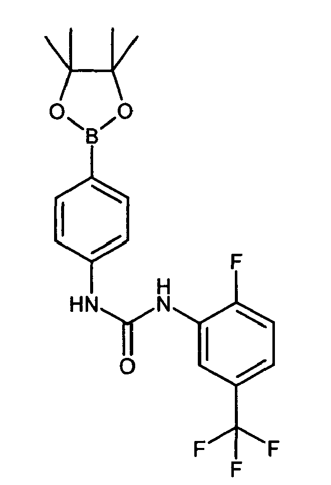

Intermediate 3 was prepared according to GP 2 from 4-(4,4,5,5-tetramethyl-1,3,2-dioxaborolan-2-yl)-aniline and (2-fluoro-5-trifluoromethyl)-phenyl isocyanate. Attempted purification by column chromatography failed. Therefore, the impure intermediate was used as is without further characterisation in the following step (see Expl. 1.5).

### Intermediate 4:

### Preparation of N-phenyl-N'-(4-(4,4,5,5-tetramethyl-1,3,2-dioxaborolan-2-yl)phenyl]urea

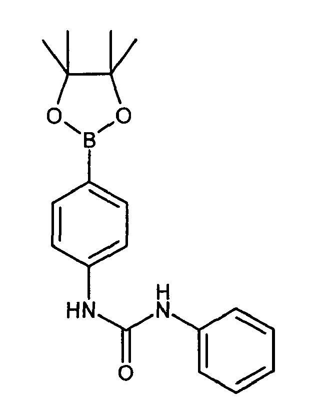

Intermediate 4 was prepared according to GP 2 from 4-(4,4,5,5-tetramethyl-1,3,2-dioxaborolan-2-yl)-aniline and phenyl isocyanate to give the slightly impure product. Yield 65 %.
MS (ESI): [M+H]⁺ = 339.

### Intermediate 5:

### Preparation of N-[4-(4,4,5,5-tetramethyl-1,3,2-dioxaborolan-2-yl)phenyl]-N'-[3-(trifluoromethyl)phenyl] urea

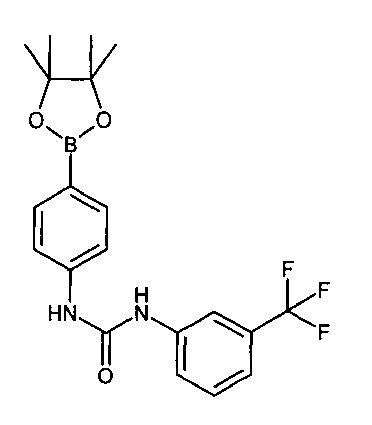

Intermediate 5 was prepared according to GP 2 from 4-(4,4,5,5-tetramethyl-1,3,2-dioxaborolan-2-yl)-aniline and 3-trifluoromethylphenyl isocyanate. Yield 89 %.
¹H-NMR (CDCl₃, 300 MHz): 7.74 (d, 2 H); 7.58 - 7.68 (m, 1 H): 7.16 - 7.55 (m, 7 H); 1.32 (s, 12 H).
MS (ESI): [M+H]⁺ = 407.

### Intermediate 6:

### Preparation of 4-methoxy-N-[4-(4,4,5,5-tetramethyl-1,3,2-dioxaborolan-2-yl)phenyl]benzenesulfonamide

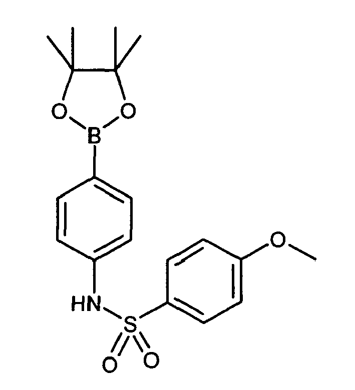

Intermediate 6 was prepared according to GP 3 from 4-(4,4,5,5-tetramethyl-1,3,2-dioxaborolan-2-yl)-aniline and 4-methoxyphenyl sulfonyl chloride. Yield 88 %.
¹H-NMR (CDCl₃, 300 MHz): 7.66 (d, 2 H); 7.61 (d, 2 H); 6.98 (d, 2 H); 6.80 (d, 2 H); 6.62 (s br, 1 H): 3.74 (s, 3 H); 1.24 (s, 12 H).
MS (ESI): [M+H]⁺ = 390.

### Preparation of example compounds

### Example 1.1

### Preparation of 2-[4-[4,4-dioxo-4λ⁶-thia-2,5,9-triaza-1 (2,4)-pyrimidina-3(1,3)-benzenacyctononaphan-1⁵-yl]phenoxy]-1-(4-fluorophenyl)ethanone

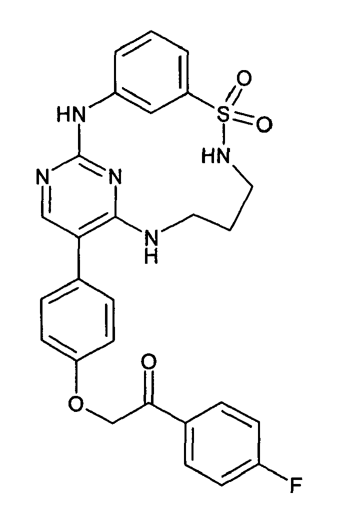

Example Compound 1.1 was prepared according to GP 4 from 1⁵-iodo-4-thia-2,5,9-triaza-1(2,4)-pyrimidina-3(1,3)-benzenacyclononaphan-4,4-dioxide and 1-(4-fluorophenyl)-2-[4-(4,4,5,5-tetramethyl-1,3,2-dioxaborolan-2-yl)phenoxy]ethanone. Yield 22 %.
¹H-NMR (DMSO, 300 MHz): 9.57 (s, 1 H); 9.45 (s, 1 H); 8.09 - 8.21 (m, 2 H); 7.70 - 7.83 (m, 2 H); 7.23 - 7.50 (m, 7 H); 7.06 (d, 2 H); 6.89 (t br, 1 H); 5.61 (s, 2 H); 3.19-3.51 (m, 4 H); 1.73 - 1.95 (m, 2 H).
MS (ESI): [M+H]⁺ = 534.

### Example 1.2

### Preparation of 2-[4-[4,4-dioxo-4λ⁶-thia-2,5,9-triaza-1(2,4)-pyrimidina-3(1,3)-benzenacyclononaphan-15 -yl]phenoxy]-1-phenylethanone

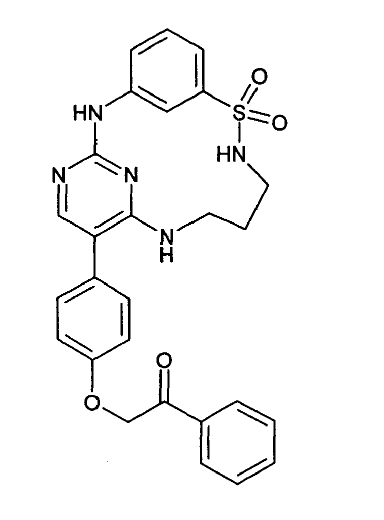

Example Compound 1.2 was prepared according to GP 4 from 1⁵-iodo-4-thia-2,5,9-triaza-1(2,4)-pyrimidina-3(1,3)-benzenacyclononaphan-4,4-dioxide and 1-phenyl-2-[4-(4,4,5,5-tetramethyl-1,3,2-dioxaborolan-2-yl)phenoxy]ethanone. Yield 9 %.
¹H-NMR (DMSO, 300 MHz): 9.58 (s, 1 H); 9.47 (s, 1 H); 8.07 (d, 2 H); 7.66-7.81 (m, 3 H); 7.60 (t, 2 H); 7.23 - 7.43 (m, 5 H); 7.04 (d, 2 H); 6.89 (t br, 1 H); 5.62 (s, 2 H); 3.18 - 3.48 (m, 4 H); 1.74 - 1.94 (m, 2 H).
MS (ESI): [M+H]⁺ = 516.

### Example 1.3

### Preparation of N-[4-[4,4-dioxo-4λ⁶ -thia-2,6,9-triaza-1(2,4)-pyrimidina-3(1,3)-benzenacyclononaphan-1⁵-yl]phenyl]-N'-phenylurea

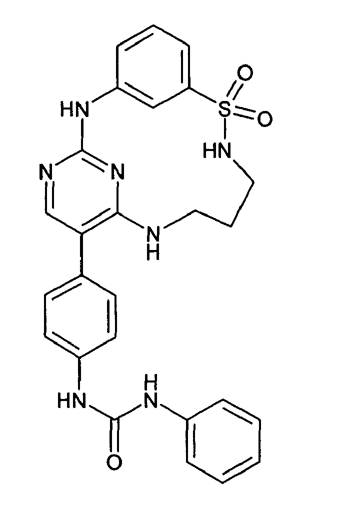

Example Compound 1.3 was prepared according to GP 4 from 1⁵-iodo-4-thia-2,5,9-triaza-1(2,4)-pyrimidina-3(1,3)-benzenacyclononaphan-4,4-dioxide and N-phenyl-*N*'-[4-(4,4,5,5-tetramethyl-1,3,2-dioxaborolan-2-yl)phenyl]urea. Yield 26 %.
¹H-NMR (DMSO, 300 MHz): 9.59 (s, 1 H); 9.47 (s, 1 H); 8.79 (s, 1 H); 8.68 (s, 1 H); 7.70 - 7.85 (m, 2 H); 7.56 (d, 2 H); 7.47 (d, 2 H); 7.20 - 7.42 (m, 7 H); 6.98 (t, 1 H); 6.92 (t br, 1 H); 3.20 - 3.50 (m, 4 H); 1.75 - 1.93 (m, 2 H).
MS (ESI): [M+H]⁺ = 516.
mp.273 °C (dec.)

### Example 1.4

### Preparation of M-[4-[4,4-dioxo-4λ⁶-thia-2,5,9-triaza-1 (2,4)-pyrimidina-3(1 ,3)-benzenacyclononaphan-1⁶-yl]phenyl]-N'-[3-(trifluoromethyl)phenyl]urea

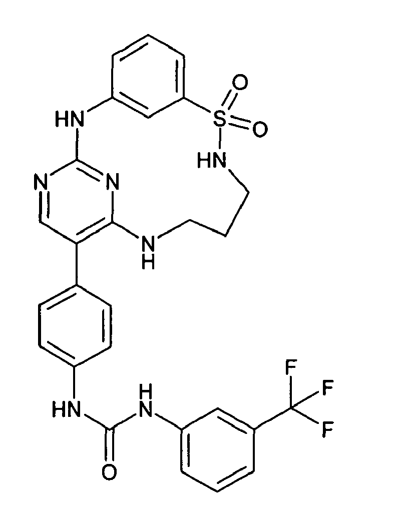

Example Compound 1.4 was prepared according to GP 4 from 1⁵-iodo-4-thia-2,5,9-triaza-1(2,4)-pyrimidina-3(1,3)-benzenacyclononaphan-4,4-dioxide and *N-*[4-(4,4,5,5-tetramethyl-1,3,2-dioxaborolan-2-yl)phenyl]-N'-[3-(trifluoromethyl)-phenyl]urea. Yield 26 %.
¹H-NMR (DMSO, 400 MHz): 9.57 (s, 1 H); 9.49 (s, 1 H); 9.05 (s, 1 H); 8.91 (s, 1 H); 8.02 (s, br, 1 H); 7.81 (s, 1 H); 7.77 (t, 1 H); 7.49 - 7.67 (m, 4 H); 7.23 - 7.42 (m, 6 H); 6.91 (t, 1 H); 3.37 - 3.52 (m, 2 H); 3.23 - 3.35 (m, 2 H); 1.78 - 1.96 (m, 2 H).
MS (ESI): [M+H]⁺ = 584.
mp. 228 °C (dec.)

### Example 1.5

### Preparation of N-[4-[4,4-dioxo-4λ⁶-thia-2,5,9-triaza-1(2,4)-pyrimidina-3(1,3)-benzenacyclononaphan-1⁵-yl]phenyl]-N'-[2-fluoro-5-(trifluoromethyl)phenyl]urea

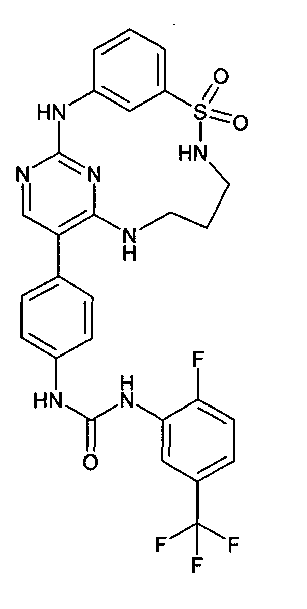

Example Compound 1.5 was prepared according to GP 4 from 1⁵-iodo-4-thia-2,5,9-triaza-1(2,4)-pyrimidina-3(1,3)-benzenacyclononaphan-4,4-dioxide and N-[2-fluoro-5-(trifluoromethyl)phenyl]-N' -[4-(4,4,5, 5-tetramethyl-1 ,3,2-dioxaborolan-2-yl)phenyl]urea. Yield 27 %.
¹H-NMR (DMSO, 400 MHz): 9.58 (s, 1 H); 9.48 (s, 1 H); 9.30 (s, 1 H); 8.90 (s, 1 H); 8.62 (d, 1 H); 7.73 - 7.82 (m, 2 H); 7.45 - 7.60 (m, 3 H); 7.22 - 7.42 (m, 6 H); 6.92 (t br, 1 H); 3.33 - 3.48 (m, 2 H); 3.20 - 3.30 (m, 2 H); 1.78 - 1.92 (m, 2 H).
MS (ESI): [M+H]⁺ = 602.
mp. 250 °C (dec.)

### Example 1.6

### Preparation of N-[4-[4,4-dioxo-4λ⁶-thia-2,6,9-triaza-1 (2,4)-pyrimidina-3(1,3)-benzenacyclononaphan-1⁵-yl]phenyl]-4-methoxybenzenesulfonamide

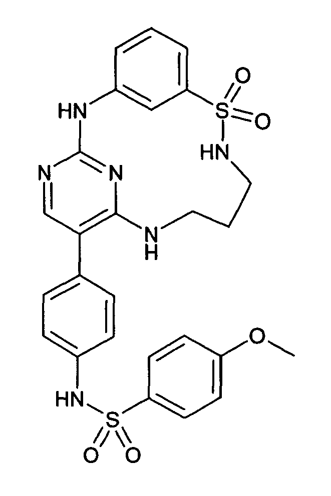

Example Compound 1.6 was prepared according to GP 4 from 1⁵-iodo-4-thia-2,5,9-triaza-1(2,4)-pyrimidina-3(1,3)-benzenacyclononaphan-4,4-dioxide and 4-methoxy-N-[4-(4,4,5,5-tetramethyl-1,3,2-dioxaborolan-2-yl)phenyl]benzenesulfonamide. Yield 16 %.
¹H-NMR (DMSO, 300 MHz): 10.32 (s, 1 H); 9.55 (s, 1 H); 9.43 (s, 1 H); 7.69 7.84 (m, 4 H); 7.33-7.42 (m, 1 H); 7.22-7.32 (m, 4 H); 7.18 (d, 2 H); 7.10 (d, 2 H); 6.86 (t br, 1 H); 3.80 (s, 3 H); 3.20 - 3.43 (m, 4 H); 1.72 - 1.88 (m, 2 H).
MS (ESI): [M+H]⁺ = 567.
mp. 192-195 °C

### Example 1.7

### Preparation of 1⁵-(1,3-benzodioxol-5-yl)-4-thia-2,5,9-triaza-1(2,4)-pyrimidina-3(1,3)-benzenacyclononaphane 4,4-dioxide

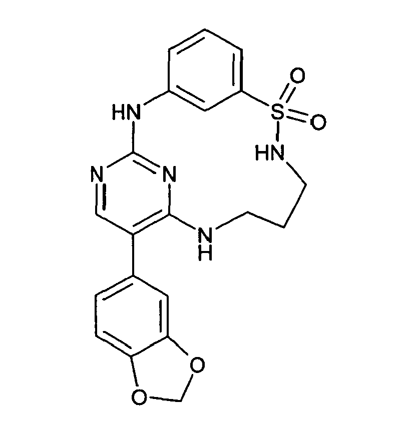

Example Compound 1.7 was prepared according to GP 4 from 1⁵-iodo-4-thia-2,5,9-triaza-1(2,4)-pyrimidina-3(1,3)-benzenacyclononaphan-4,4-dioxide and 3,4-(methylenedioxy)phenylboronic acid. Yield 51 %.
¹H-NMR (DMSO, 400 MHz): 9.56 (s, 1 H); 9.43 (s, 1 H) 7.71 - 7.78 (m, 2 H); 7.39 (m, 1 H); 7.25 - 7.34 (m, 2 H); 7.00 (d, 1 H); 6.94 (s, 1 H); 6.89 (t, 1 H); 6.83 (dd, 1 H); 6.05 (s, 2 H); 3.33 - 3.46 (m, 2 H); 3.22 - 3.33 (m, 2 H); 1.76 - 1.89 (m, 2 H).
MS (ESI): [M+H]⁺ = 426.
mp. 340-344 °C

The following Example Compounds may be obtained using the methods described before or by standard procedures known to those skilled on the art:

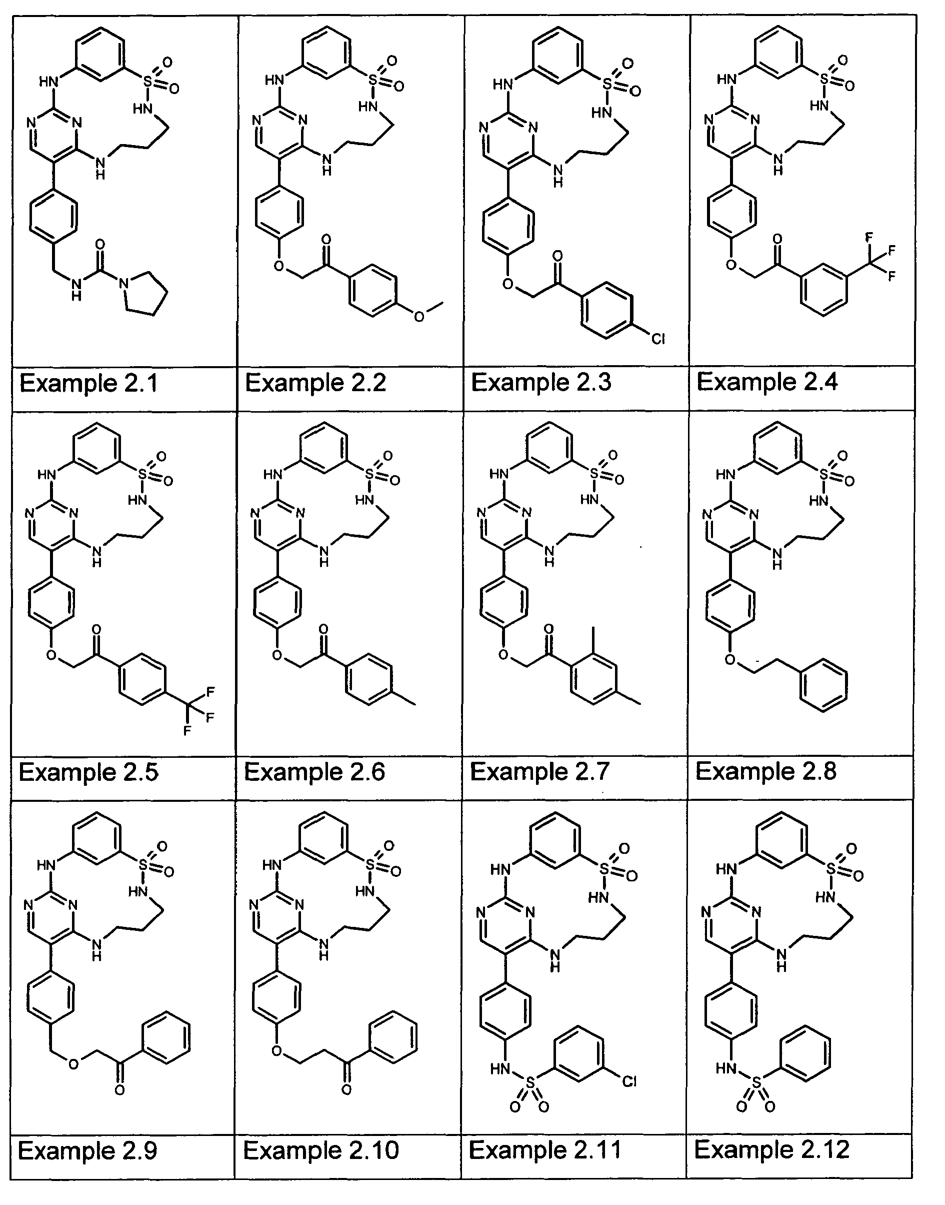

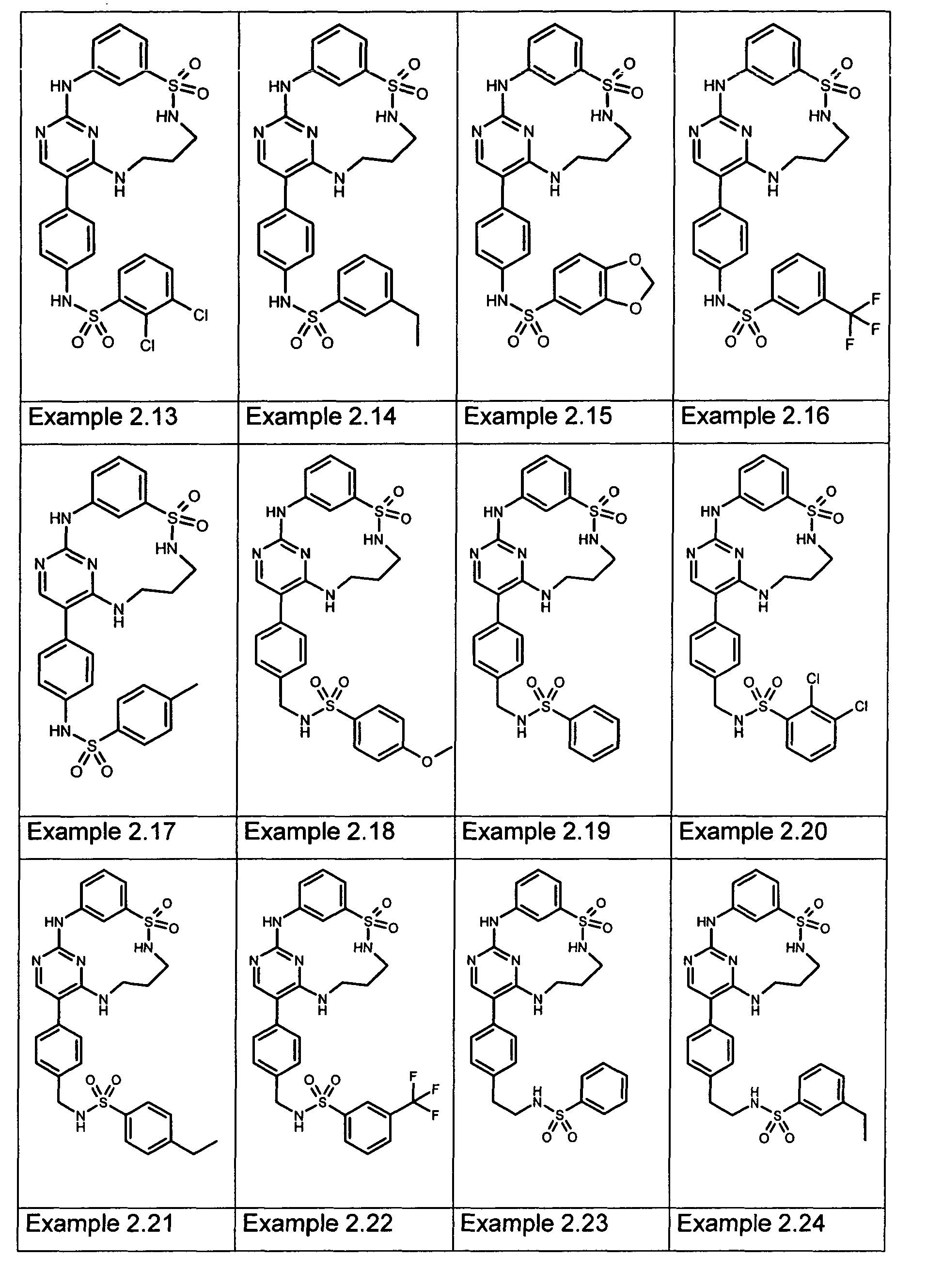

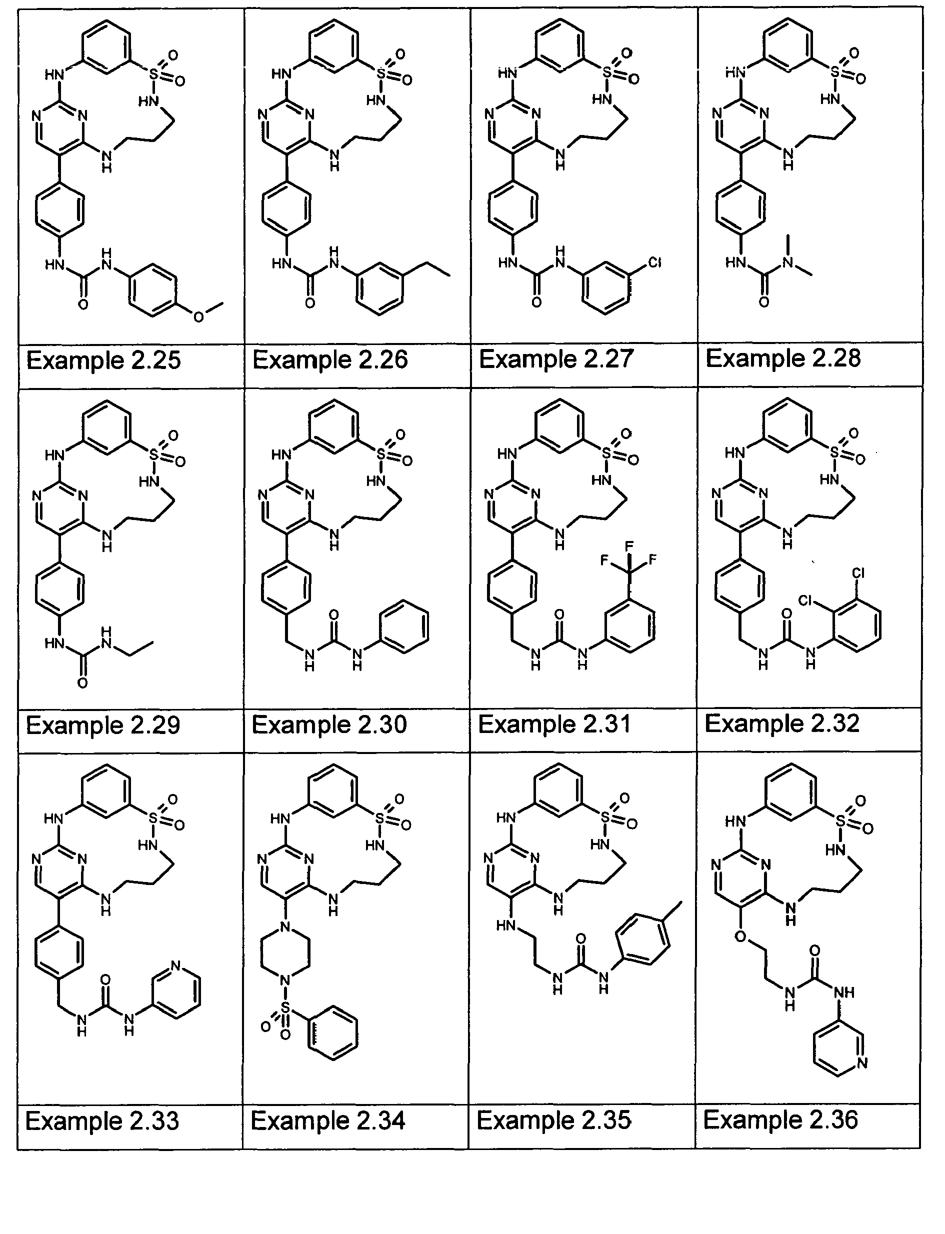

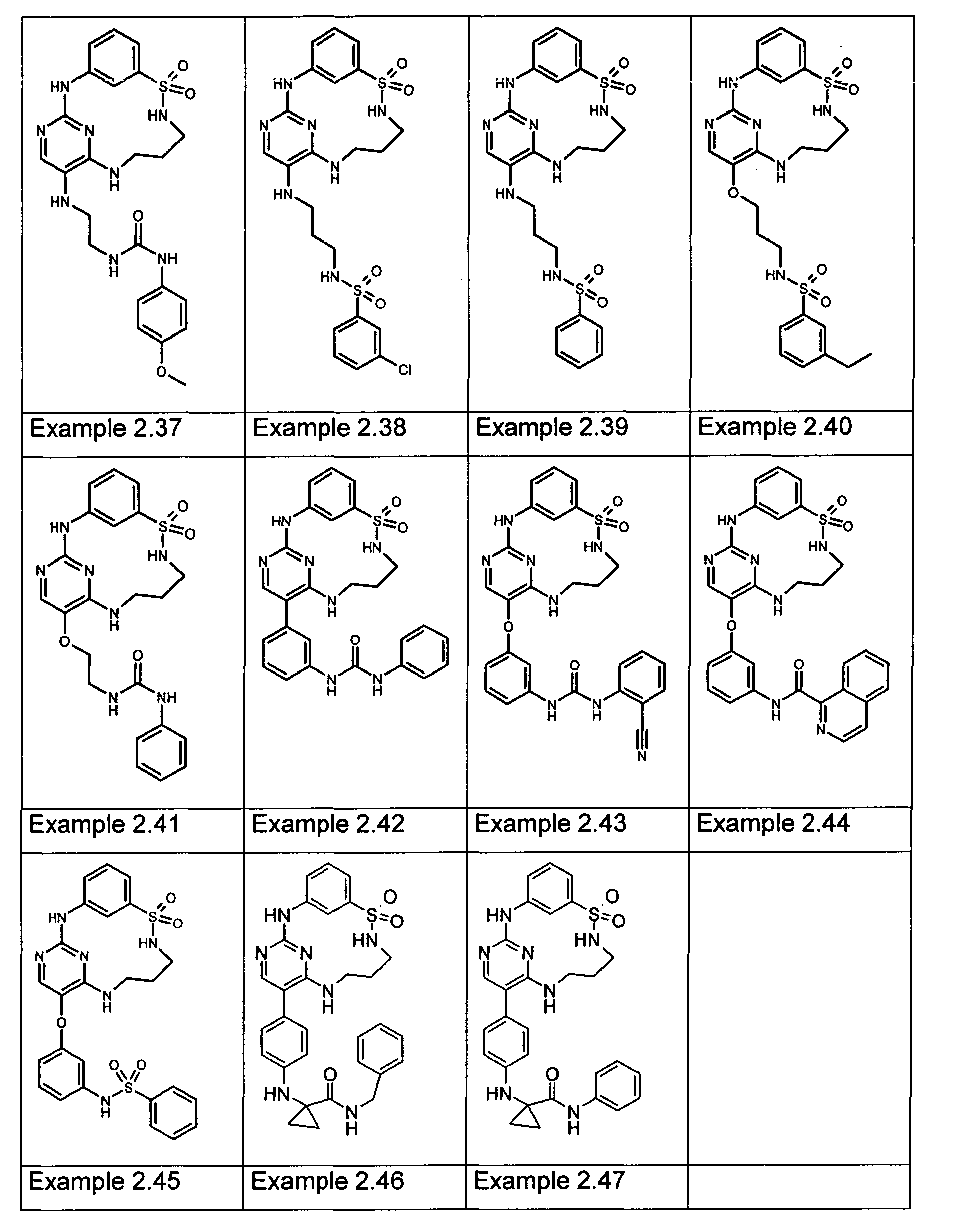

### Biological experiment 1: ELISA method

To prove the high potency activity as inhibitors of Tie2 kinase and Tie2 autophosphorylation the following ELISA-method was established and used.

Herein CHO cell-cultures, which are stably transfected by known techniques with Tie2 using DHFR deficiency as selection marker, are stimulated by angiopoietin-2. The specific autophosphorylation of Tie2 receptors is quantified with a sandwich-ELISA using anti-Tie2 antibodies for catch and anti-phosphotyrosine antibodies coupled to HRP as detection.

### Materials:

96well tissue culture plate, sterile, Greiner
96well FluoroNunc plate MaxiSorp Surface C, Nunc
96well plate polypropylene for compound dilution in DMSO
CHO Tie2/DHFR (transfected cells)
PBS-; PBS++, DMSO
MEM alpha Medium with Glutamax-I without Ribonucleosides and Deoxyribonucleosides (Gibco #32561-029) with 10% FCS after dialysis! and 1% PenStrep
Lysis buffer: 1 Tablet "Complete" protease inhibitor 1 cap Vanadate (1 mL > 40 mg/mL; working solution 2 mM) ad 50 mL with Duschl-Puffer
   pH 7.6
Anti-TIE-II antibody 1 : 425 in Coating Buffer pH 9.6
   Stock solution: 1.275 mg/mL > working.: 3 µg/mL
PBST:2 bottles PBS(10x) + 10ml Tween, fill up with VE-water
RotiBlock 1 : 10 in VE-water
Anti-Phosphotyrosine HRP-Conjugated 1 : 10000 in 3% TopBlock 3% TopBlock in PBST
BM Chemiluminescence ELISA Substrate (POD)
   solution B 1 : 100 solution A
SF9 cell culture medium
Ang2-Fc in SF9 cell culture medium

Cell experiment:
Dispense 5 x 10⁴ cells / well /98 µL in 96well tissue culture plate Incubate at 37 °C / 5% CO₂
After 24 h add compounds according to desired concentrations
Add also to control and stimulated values without compounds 2 µL DMSO
And mix for a few min at room temperature
Add 100 µL Ang2-Fc to all wells except control, which receives insect medium
Incubate 20 min at 37 °C.
Wash 3x with PBS++
Add 100 µl Lysis buffer / well and shake a couple of min at room temperature
Store lysates at 20 °C before utilizing for the ELISA

Performance of sandwich-ELISA
Coat 96well FluoroNunc Plate MaxiSorp Surface C with anti-Tie2 Mab 1 : 425 in Coating buffer pH 9.6; 100 µL / well overnight at 4 °C
Wash 2x with PBST
Blcock plates with 250 µL / well RotiBlock 1 : 10 in VE-water
Incubate for 2 h at room temperature or overnight at 4 °C shaking
Wash 2x in PBST
Add thawed lysates to wells and incubate overnight shaking at 4 °C
Wash 2x with PBST
Add 100 µL /well anti-Phosphotyrosine HRP-Conjugated 1 : 10000 in 3% TopBlock (3% TopBlock in PBST) and incubate overnight under shaking
Wash 6x with PBST
Add 100 µL/well BM Chemiluminescence ELISA Substrate (POD) solutions 1 und 2 (1 : 100)
Determine luminescence with the LumiCount.

### Biological experiment 2: Tie-2-Kinase HTRF-Assay

To prove the effectiveness of the compound according to the present invention a Tie-2-Kinase HTRF-Assay was established.

Tie-2 phosphorylates tyrosine residues of the artificial substrate polyGAT (biotinylated polyGluAlaTyr). Detection of phosphorylated product is achieved specifically by a trimeric detection complex consisting of the phosphorylated substrate, streptavidin-XLent (SA-XLent) which binds to biotin, and Europium Cryptate-labeled anti-phosphotyrosine antibody PT66 which binds to phosphorylated tyrosine. Excitation of Europium fluorescence with 337 nm light results in emission of long-lived light with 620 nm. In case a trimeric detection complex has formed, part of the energy will be transferred to the SA-XLent fluorophore that itself then emits long-lived light of 665 nm (FRET: fluorescence resonance energy transfer). Unphosphorylated substrate does not give rise to light emission at 665nm, because no FRET-competent trimeric detection complex can be formed. Measurement is performed in a Packard Discovery or BMG Rubystar instrument. A-counts (emission at 665 nm) will be divided by B-counts (emission at 620 nm) and multiplicated with a factor of 10000. The resulting numbers are called the "well ratio" of the sample.

### Material:

Enzyme: Tie-2-Kinase, in house, aliquots (12 x 10 mL) stored at -80 °C Substrate: PolyGAT labeled with Biotin (1000 µg / mL); CIS Bio ; # 61GATBLB; aliquots stored at -20 °C
ATP: Amersham Pharmacia Biotech Inc. # 27-2056-01; 100 mM; stored at -20 °C
Antibody: PT66-Eu Cryptate ; CIS Bio ; # 61T66KLB ; 30µg/mL; aliquots stored at -20 °C
SA-XLent ; CIS Bio; # 611SAXLB ; 1000 µg/mL; aliquots stored at -80 °C Microplates : 384 Well black, SV, Greiner, # 784076

### Solutions:

### Assay buffer:

50 mM HEPES (pH 7.0), 25 mM MgCl₂, 5 mM MnCl₂, 1 mM DTT, 0.5 mM Na₃VO₄, 0.01 % (v/v) NP40, 1 x Complete EDTA free

### Enzyme working solution:

Tie-2 stock solution is diluted 1:250 in assay buffer

### Substrate working solution:

PolyGAT (1000 µg/mL; 36.23 µM) is diluted 1:90.6 to 400 nM or 77.3 ng/well, ATP (100 mM) is diluted 1 : 5000 to 20.0 µM. Both dilutions in assay buffer. Final assay concentrations: poly-GAT: 200 nM or 5.25 µg/mL, ATP: 10 µM (1 x Km each).

Detection solution: 50 mM HEPES (pH 7.0), BSA 0.2%, 0.6 M KF, 200 mM EDTA, PT66-Europium Cryptate 2.5 ng/well, SA-XLent Cis Bio 90 ng/well.

### Assay steps

All steps at 20 °C
1. 0.75 µL of compound solution in 30 % (v/v) DMSO
2. add 7 µL of substrate working solution
3. add 7 µL of enzyme working solution
4. incubate 75 min (reaction volume: 14.75 µL)
5. add 8 µL of detection solution
6. incubate 180 min or over night at 4 °C (total volume: 22.75 µL)
7. measure HTRF in Packard Discovery or BMG Rubystar instrument (delay 50 µs, integrated time 400 µs)

Final concentrations (in 14.75 µL reaction volume):
Enzyme: unknown
polyGAT (1 x Km): 200 nM (77.3 ng)
ATP (1 x Km): 10 µM
DMSO: 1.5 % (v/v)
Buffer conditions: 50 mM HEPES (pH 7.0), 25 mM MgCl₂, 5 mM MnCl₂, 1 mM DTT, 0.5 mM NaVO4, 0.01 % (v/v) NP40, 1 x Complete

### Controls:

C₀: uninhibited reaction (DMSO only)
Cᵢ: inhibited reaction with 20 µM Staurosporine

### Biological experiment 3: Proliferation test

To examine cell toxicity a cell proliferation test were established.

With the cell proliferation test different tumour cell lines (e.g. Du 145) can be examined. The cells were dispensed in RPMI 1640 culture medium, supplied with 10 % (v/v) fetal calf serum plus 1 % (v/v) Penicillin/Streptomycin solution at a cell density of 2.000 cell/100µL medium/per well (96well plate). After three hours the cells were washed with PBS (containing calcium and magnesium). 100 µl of culture medium above with 0.1 % (v/v) fetal calf serum was added and cultured at 37°C and 5% CO₂-atmosphere. Next day compounds of the present invention diluted in DMSO for appropriate concentrations were added and further 100 µL culture medium 0.5 % (v/v) fetal calf serum. After 5 days cell culturing at 37°C and 5% CO₂-atmosphere cells were washed with PBS. 20 µL of glutaraldehyde solution (11 % (v/v)) is added and the cells were slightly shaken at room temperature for 15 min. After that the cell were washed 3 times and dried in the air. 100 µL of crystal violet solution (0.1 % at pH 3.5) were added and the cells were shaken for 30 min. The cells were washed with tap water and air-dried. The colour is dissolved with 100 µL of acetic acid (10 % (v/v)) under strong shaking for 5 min. The absorption was measured at 595 nm wavelength.

The biological experiments show that the compounds presented in this application have high potency activity as inhibitors of Tie2 kinase and Tie2 autophosphorylation as measured with the ELISA-method. The IC50 values are below 1 µM. At the same time the toxicity of the compounds is well above 1 µM which is different to other compounds in this structure class, where the toxicity to tumour cell lines is such, that the IC50 values below 1 µM are observed.

Certain compounds of the invention have been found as potent inhibitors of Tie2. More specifically, the synthesized example compounds 1.1 to 1.7 throughout inhibit Tie2 with an IC50 of 1 µM or less either in the Tie2 kinase assay or in the Tie2 autophosphorylation ELISA test. While featuring high inhibitory potency against Tie2 kinase activity, certain compounds of the invention have been found to be particularly weakly cytotoxic or non-cytotoxic.

The compounds of the present invention are therefore preferentially active as antiangiogenesis inhibitors and not as cytostatic or cytotoxic agents that affect tumour cells and other proliferating tissue cells directly.

## Claims

1. A compound of the general Formula I: 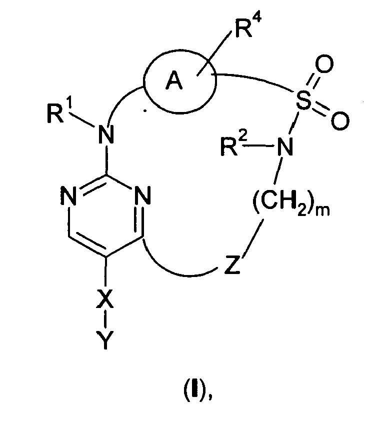 wherein
A is phenylene or C₆-heteroarylene;
Z is selected from the group comprising O, S, NR³ and CHR³;
R¹, R² and R³ are the same or different and are independently from each other selected from the group comprising hydrogen and -C₁-C₁₀-alkyl, wherein -C₁-C₁₀-alkyl is unsubstituted or singly or multiply substituted with hydroxy;
R⁴ is selected from the group comprising hydrogen, halogen, nitro, amino, cyano, -C₁-C₆-alkyl, -C₁-C₆-alkoxy, -NH-C₁-C₆-alkyl, -N(C₁₋C₆-alkyl)₂, -(CH₂)ₚ-COR⁵, -(CH₂)ₚ-NHCOR⁵, -(CH₂)ₚ-NH-CO-NR⁵R⁶, -(CH₂)ₚ-NHS(O)₂R⁵, -(CH₂)ₚ-CO-NR⁵R⁶ and -O-(CH₂)ₚ₋COR⁵,
X is a bond or methylene;
Y is selected from the group comprising methylenedioxyphenyl, ethylenedioxyphenyl, -phenylene-D-NH-COR⁵, -phenylene-D-NH-CONR⁵R⁶, -phenylene-D-NH-S(O)₂R⁵, -phenylene-D-O-(CH₂)ₚ₋COR⁵, -phenylene-D-O-(CH₂)ₚ-R⁵, -N-(R⁵-A)-piperazin-N'- ylmethyl, -N-(R⁷-SO₂-)-piperazin-N'-yl, -OXY-C₆-C₁₈-aryl, -oxy-C₅₋C₁₈-heteroaryl, -oxy-(CH₂)ₙ-NH-COR⁵,-oxy-(CH₂)ₙ-NH-CONR⁵R⁵ -oxy-(CH₂)ₙ-NH-S(O)₂R⁵, -NH-(CH₂)ₙ-NH-COR⁵, -NH-(CH₂)ₙ-NH-CONR⁵R⁶, -NH-(CH₂)ₙ-NH- S(O)₂R⁵ and -phenylene-NH-E-CONR⁵R⁶, wherein phenylene is unsubstituted or singly or multiply substituted independently from each other with hydroxy, halogen, nitro, cyano, carboxy, amino, -C₁-C₆-alkyl, -C₁-C₆-alkoxy, -NH-C₁₋C₆-alkyl, -N(C₁-C₆-alkyl)₂, -C₁-C₆-halogenalkyl, -C₁-C₆₋halogenalkoxy, -C₁-C₆-alkylthio and/or -C₁-C₆-alkylcarbonyl, and wherein -oxy-C₆-C₁₈-aryl and -oxy-C₅-C₁₈-heteroaryl are unsubstituted or singly or multiply substituted independently from each other with hydroxy, halogen, nitro, cyano, carboxy, amino, -C₁-C₆-alkyl, -C₁-C₆-alkoxy, NH-C₁-C₆-alkyl, N(C₁-C₆-alkyl)2, -C₁₋C₆-haloalkyl, C₁-C₆-haloalkoxy, C₁-C₆-alkylthio, C₁-C₆₋alkylcarbonyl, -NH-S(O)₂-R⁵, -NH-COR⁵, -NHCONR⁵R⁶, -O-(CH₂)ₚ-COR⁵ and/or -O-(CH₂)ₚ-R⁵,
A is -SO₂-, -CONH- or -CO-;
D is a bond, methylene or ethylene;
E is -CR⁸R⁹-, wherein R⁸ and R⁹ are the same or different and are independently from each other selected from the group comprising hydrogen and methyl; or R⁸ and R⁹ together form a 3- to 10-membered methylene tether, in which up to two methylene groups are optionally replaced by O, S and/or NR¹;
R⁵ is selected from the group comprising hydrogen and residues selected from the group comprising -C₁-C₆-alkyl, -C₂-C₆-alkenyl, -C₂-C₆-alkinyl, -C₃-C₈-cycloalkyl, -(CH₂)ₚ-C₆-C₁₁-aryl and -(CH₂)ₚ₋C₅-C₁₀-heteroaryl, wherein said residues are unsubstituted or singly or multiply substituted independently from each other with hydroxy, halogen, nitro, cyano, carboxy, amino, -C₁-C₆-alkyl, -C₁₋C₆-alkenyl, -C₁-C₆-alkinyl, -C₁-C₆-alkoxy, -NH-C₁-C₆-alkyl, -N(C₁₋C₆-alkyl)₂, -C₁-C₆-halogenalkyl, -C₁-C₆-halogenalkoxy, -C₁-C₆₋alkylthio, -S(O)-C₁-C₆-alkyl, -S(O)₂-C₁-C₆-alkyl, -C₁-C₆₋alkylcarbonyl, phenyl, phenoxy and/or pyridyl, and wherein the C-atoms of the C -backbone of -C₃-C₈-cycloalkyl are uninterrupted or singly or multiply interrupted by nitrogen-atoms, oxygen-atoms, sulphur-atoms and/or one or more C=O-moieties and/or wherein one or more double bonds may be contained in the C-backbone; or R⁵ and R⁶ together form a 3- to 10-membered methylene tether, in which up to two methylene groups may be replaced by O, S and/or -NR¹;
R⁶ is hydrogen or -C₁-C₁₀-alkyl, or R⁵ and R⁶ together form a 3- to 10-membered methylene tether, in which up to two methylene groups may be replaced by O, S and/or NR¹;
R⁷ is -C₆-C₁₁-aryl or -C₅-C₁₀-heteroaryl, wherein -C₆-C₁₁-aryl or -C₅₋C₁₀-heteroaryl are unsubstituted or singly or multiply substituted with hydroxy, halogen, nitro, cyano, carboxy, amino, -C₁-C₆-alkyl, -C₁-C₆-alkoxy, -NH-C₁-C₆-alkyl, -N(C₁-C₆-alkyl)₂, -C₁-C₆-haloalkyl, -C₁-C₆-haloalkoxy, -C₁-C₆-alkylthio, -S(O)-C₁-C₆-alkyl, -S(O)₂-C₁₋C₆-alkyl, -C₁-C₆-alkylcarbonyl, phenyl, phenoxy and/or pyridyl;
m is from 3 to 6;
n is 2 or 3;
p is from 0 to 2; and
solvates, hydrates, N-oxides, isomers, diastereomers, enantiomers and salts thereof.

2. The compound according to claim 1 or 2, wherein
A is phenylene;
Z is -NR³; and
m is 3.

3. The compound according to claim 1 or 2, wherein
X is a bond and
Y is methylenedioxyphenyl or ethylenedioxyphenyl.

4. The compound according to claim 1 or 2, wherein
X is a bond and
Y is -phenylene-D-NH-COR⁵.

5. The compound according to claim 1 or 2, wherein
X is a bond and
Y is -phenylene-D-NH-CONR⁵R⁶.

6. The compound according to claim 1 or 2, wherein
X is a bond and
Y is -phenylene-D-NH-S(O)₂R⁵

7. The compound according to claim 1 or 2, wherein
X is a bond and
Y is -phenylene-D-O-(CH₂)ₚ-COR⁵.

8. The compound according to claim 7, wherein
D is bond or methylene

9. The compound according to claim 1 or 2, wherein
X is a bond and
Y is -phenylen-NH-E-CONR⁵R⁶.

10. The compound according to claim 1 or 2, wherein
X is a bond and
Y is -oxy-C₆-C₁₈-aryl, wherein -oxy-C₆-C₁₈-aryl is unsubstituted or singly or multiply substituted independently from each other with hydroxy, halogen, nitro, cyano, carboxy, amino, -C₁-C₆-alkyl, -C₁₋C₆-alkoxy, -NH-C₁-C₆-alkyl, -N(C₁-C₆-alkyl)₂, -C₁-C₆-haloalkyl, -C₁₋C₆-haloalkoxy, -C₁-C₆-alkylthio, -C₁-C₆-alkylcarbonyl, -NH-S(O)₂₋R⁵, -NH-COR⁵, -NHCONR⁵R⁶, -O-(CH₂)_{P}-COR⁵ and/or-O-(CH2)p-R⁵

11. The compound of claim 10, wherein -C₆-C₁₈-aryl is phenyl.

12. The compound of claim 10 or 11, wherein phenyl is substituted with -NH-COR⁵ or -NH-CONHR⁵.

13. The compound according to claim 1 or 2, wherein
X is a bond or methylene and
Y is -N-(R⁵-A)-piperazin-N'-ylmethyl.

14. The compound according to claim 1 or 2, wherein
X is a bond or methylene and
Y is -N-(R⁷-SO₂)-piperazin-N'-yl.

15. The compound according to claim 1 or 2, wherein
X is a bond and
Y is -oxy-(CH₂)ₙ-NH-COR⁵

16. The compound according to claim 1 or 2, wherein
X is a bond and
Y is -oxy-(CH₂)ₙ-NH-CONR⁵R⁶.

17. The compound according to claim 1 or 2, wherein
X is a bond and
Y is -oxy-(CH₂)ₙ-NH-S(O)₂R⁵,

18. The compound according to claim 1 or 2, wherein
X is a bond and
Y is -NH-(CH₂)ₙ-NH-COR⁵.

19. The compound according to claim 1 or 2, wherein
X is a bond and
Y is -NH-(CH₂)ₙ-NH-CONR⁵R⁶.

20. The compound according to claim 1 or 2, wherein
X is a bond and
Y is -NH-(CH₂)ₙ-NH- S(O)₂R⁵.

21. A method of preparing the compound according to any one of claims 1 - 20, wherein the method comprises the following method step: 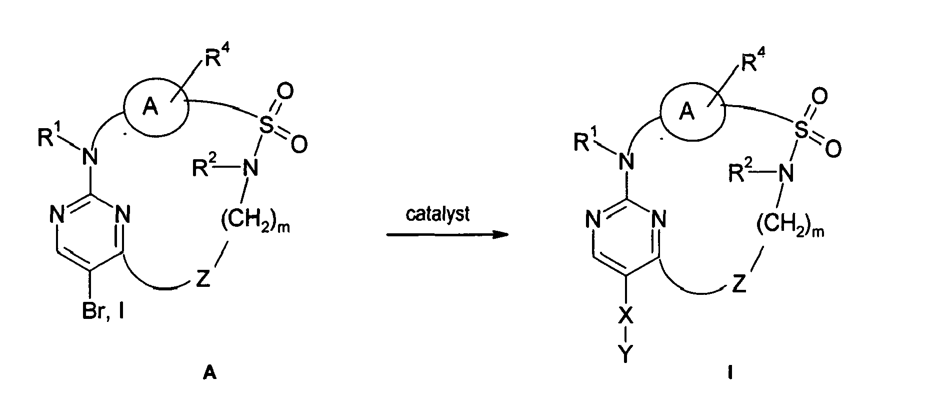 wherein R¹, R², R⁴, A, Z and m in the macrocycle A have the same meaning as in Formula 1, wherein in Formula I X is a bond and Y is selected from the group comprising -phenylene-D-NH-COR⁵, - phenylene-D-NH-CONR⁵R⁶, -phenylene-D-NH-S(O)₂R⁵, - phenylene-D-O-(CH₂)ₚ-COR⁵, -oxy-C₆-C₁₁-aryl, or -OXY-C₅-C₁₀₋heteroaryl with the meaning above.

22. A method of preparing the compound according to any one of claims 1 20, wherein the method comprises the following method step: 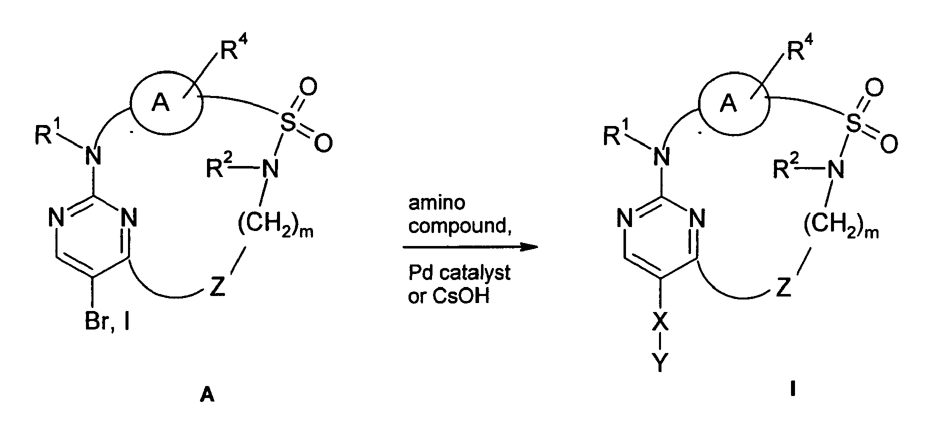 wherein R¹, R², R⁴, A, Z and m in the macrocycle A have the same meaning as in Formula I, wherein in Formula I X is a bond and Y is selected from the group comprising -NH-(CH₂)ₙ-COR⁵-, -NH-(CH₂)ₙ-CONR⁵R⁶, or -NH-(CH₂)ₙ-S(O)₂R⁵ with the meaning above.

23. A pharmaceutical composition which comprises the compounds of the Formula ! according to any one of claims 1 to 20 or a pharmaceutically acceptable salt or an *in vivo* hydrolysable ester thereof, and a pharmaceutically-acceptable diluent or carrier.

24. A use of the compound of any one of claims 1 to 20 for manufacturing a pharmaceutical composition for the treatment of diseases of dysregulated vascular growth or of diseases which are accompanied with dysregulated vascular growth.

25. The use according to claim 24, wherein the diseases are retinopathy, other angiogenesis dependent diseases of the eye, rheumatoid arthritis, and other inflammatory diseases associated with angiogenesis.

26. The use of claim 25, wherein the angiogenesis dependent diseases of the eye are cornea transplant rejection, age-related macular degeneration.

27. The use of claim 25, wherein the inflammatory diseases associated with angiogenesis are psoriasis, delayed type hypersensitivity, contact dermatitis, asthma, multiple sclerosis, restenosis, pulmonary hypertension, stroke, and diseases of the bowel.

28. The use according to claim 24, wherein the diseases are coronary and peripheral artery disease.

29. The use according to claim 24, wherein the diseases are ascites, edema like brain tumour associated edema, high altitude trauma, hypoxia induced cerebral edema pulmonary edema and macular edema or edema following burns and trauma, chronic lung disease, adult respiratory distress syndrome, bone resorbtion and for benign proliferating diseases like myoma, benign prostate hyperplasia and wound healing for the reduction of scar formation, reduction of scar formation scar formation during regeneration of damaged nerves, endometriosis, pre-eclampsia, postmenopausal bleeding and ovarian hyperstimulation.

30. The use according to claim 24, wherein the diseases is a solid tumour and/or metastases thereof.

31. A method for treating a disease of dysregulated vascular growth or diseases which are accompanied with dysregulated vascular growth by using of the compound of any one of claims 1 to 20.

32. The method according to claim 31, wherein the diseases are retinopathy, other angiogenesis dependent diseases of the eye rheumatoid arthritis, and other inflammatory diseases associated with angiogenesis.

33. The method according to claim 32, wherein the angiogenesis dependent diseases of the eye are cornea transplant rejection, age-related macular degeneration.

34. The method according to 32, wherein the inflammatory diseases associated with angiogenesis are psoriasis, delayed type hypersensitivity, contact dermatitis, asthma, multiple sclerosis, restenosis, pulmonary hypertension, stroke, and diseases of the bowel.

35. The method according to claim 31, wherein the diseases are coronary and peripheral artery disease.

36. The method according to claim 31, wherein the diseases are ascites, edema like brain tumour associated edema, high altitude trauma, hypoxia induced cerebral edema pulmonary edema and macular edema or edema following burns and trauma, chronic lung disease, adult respiratory distress syndrome, bone resorbtion and for benign proliferating diseases like myoma, benign prostate hyperplasia and wound healing for the reduction of scar formation, reduction of scar formation scar formation during regeneration of damaged nerves, endometriosis, pre-eclampsia, postmenopausal bleeding and ovarian hyperstimulation.

37. The method according to claim 31, wherein the diseases is a solid tumour and/or metastases thereof.
